(19) Europäisches Patentamt
European Patent Office
Office européen des brevets

(11) Veröffentlichungsnummer: **0 323 799 B1**

(12) **EUROPÄISCHE PATENTSCHRIFT**

(45) Veröffentlichungstag der Patentschrift: **11.11.92**

(51) Int. Cl.5: **C07D 413/04**, C07D 233/90, C07D 413/14, C07D 401/12, A61K 31/415, A61K 31/41, A61K 31/44, A61K 31/335

(21) Anmeldenummer: **88730278.4**

(22) Anmeldetag: **14.12.88**

Die Akte enthält technische Angaben, die nach dem Eingang der Anmeldung eingereicht wurden und die nicht in dieser Patentschrift enthalten sind.

# (54) Imidazolderivate II.

(30) Priorität: **14.12.87 DE 3742716**

(43) Veröffentlichungstag der Anmeldung: **12.07.89 Patentblatt 89/28**

(45) Bekanntmachung des Hinweises auf die Patenterteilung: **11.11.92 Patentblatt 92/46**

(84) Benannte Vertragsstaaten: **AT BE CH DE ES FR GB GR IT LI LU NL SE**

(56) Entgegenhaltungen:
**EP-A- 0 185 961**
**EP-A- 0 284 277**
**WO-A-88/01268**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Biere, Helmut, Dr.**
**Zeltinger Strasse 15**
**W-1000 Berlin 28(DE)**
Erfinder: **Rohde, Ralph, Dr.**
**Schwatlostrasse 14**
**W-1000 Berlin 45(DE)**
Erfinder: **Schneider, Herbert Hans, Dr.**
**Duisburger Strasse 20**
**W-1000 Berlin 15(DE)**
Erfinder: **Turski, Lechoslaw, Dr.**
**Spandauer Strasse 54**
**W-1000 Berlin 20(DE)**

**EP 0 323 799 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue ZN-aktive Imidazolderivate der allgemeinen Formel I

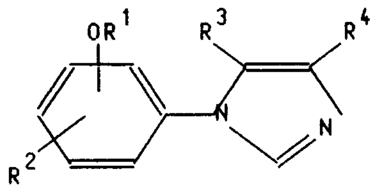

(I)

worin

$R^1$ Wasserstoff, einen gegebenenfalls ein- bis dreifach mit Halogen, Nitro, Cyano, Hydroxy, Mercapto, $C_{1-4}$-Alkyl, $C_{1-4}$-Alkoxy, $C_{1-4}$-Alkylthtio, $C_{1-4}$-Alkylsulfinyl, $C_{1-4}$-Alkylsulfonyl oder einer gegebenenfalls mit $C_{1-4}$-Alkyl, $C_{1-4}$-Alkanoyl oder Sulfonyl mono- oder disubstituierten Aminogruppe substituierten $C_{1-10}$-Kohlenwasserstoff- oder 5-6 gliedrigen Hetarylrest, der ein bis zwei Schwefel-, Stickstoff- und/oder Sauerstoffatome enthält, oder einen 5-6-gliedrigen cyclischen Etherrest bedeutet,

$R^2$ Wasserstoff, Halogen, eine gegebenenfalls mit $C_{1-4}$-Alkyl, $C_{1-4}$-Alkanoyl oder Sulfonyl mono- oder disubstituierte Aminogruppe, eine Nitro-, Azid-, Thiocyanat- oder Cyanogruppe, einen gegebenenfalls mit Halogen substituierten geraden oder verzweigten $C_{1-10}$-Alkylrest oder -$OR^1$ mit $R^1$ in der vorne genannten Bedeutung und

$R^1$ und $R^2$ gemeinsam mit dem Sauerstoffatom einen gesättigten oder ungesättigten 5- bis 7-gliedrigen Ring bilden, der ein weiteres Sauerstoffatom enthalten kann,

$R^3$ Wasserstoff, eine gerade oder verzweigte $C_{1-6}$-Alkylgruppe oder eine $C_{1-6}$-Alkoxyalkylgruppe,

$R^4$ -$COOR^5$, -$CONR^6R^7$, -CN

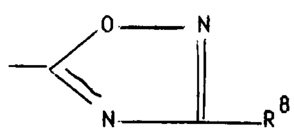

oder

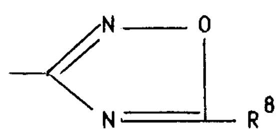

bedeuten,

mit $R^5$ in der Bedeutung von Wasserstoff, einer geraden oder verzweigten $C_{1-6}$-Alkylgruppe, $R^6$ und $R^7$ sind gleich oder verschieden und stellen Wasserstoff oder eine gerade, verzweigte oder cyclische Alkylgruppe mit bis zu 7 Kohlenstoffatomen dar oder bilden gemeinsam mit dem Stickstoffatom gegebenenfalls mit ein bis zwei $C_{1-4}$-Alkyl substituiertes Morpholin, Piperazin, Pyrrolidin, Piperidin oder Thiomorpholin und $R^8$ in der Bedeutung von Wasserstoff oder einer geraden, verzweigten oder cyclischen Alkylgruppe mit bis zu 7 Kohlenstoffatomen,

Die Substituenten am Phenylrest können in o-, m- oder p-Stellung stehen, wobei der Rest $R^2$ insbesondere ein- bis zweifach auftreten kann und die Reste $R^2$ und -$OR^1$ gleich oder verschieden sein können. Unter Halogen ist jeweils Fluor, Chlor, Brom oder Jod zu verstehen.

Als Kohlenwasserstoffrest $R^1$ kommen gesättigte oder ungesättigte, geradkettige oder verzweigte gegebenenfalls substituierte Alkylgruppen mit vorzugsweise 1 bis 6 Kohlenstoffatomen in Betracht, ferner gesättigte oder ungesättigte Cycloalkyl- oder Cycloalkylalkylgruppen mit vorzugsweise 3 - 7 Kohlenstoffatomen, wobei eine $CH_2$-Gruppe gegebenenfalls durch ein Sauerstoffatom ersetzt sein kann, sowie gegebenenfalls substituierte Aryl- oder Aralkylgruppen mit maximal 10 Kohlenstoffatomen.

Als gesättigte, geradkettige oder verzweigte Alkylreste sind jeweils die niederen Alkylreste wie Methyl, Ethyl, Propyl, i-Propyl, Butyl, sek. Butyl, Isobutyl, tert. Butyl sowie Pentyl, Hexyl, 2-Methylbutyl, 2,2-Dimethylpropyl gemeint.

Als ungesättigte Alkylgruppen seien als bevorzugt die folgenden Alkenyl- und Alkinylreste genannt: 1-Propenyl, 2-Propenyl, 3-Methyl-2-propenyl, 2-Propinyl.

Als Substituenten der Alkylgruppe kommen Halogene wie insbesondere Fluor, Chlor und Brom, Hydroxy-, $C_{1-4}$-Alkoxy- und Aminogruppen, die gegebenenfalls auch durch niedere Alkylgruppen mono-

oder di-substituiert sein können, in Betracht, Liegt als Substituent Fluor vor, so ist die Perfluoralkyl-Verbindung als bevorzugt anzusehen.

Unter Cycloalkylresten sind jeweils gesättigte Reste wie beispielsweise Cyclopropyl, Cyclobutyl, Cyclopentyl, Cyclohexyl, Cycloheptyl zu verstehen sowie ungesättigte Reste wie beispielsweise Cyclopentenyl.

Bedeutet der Kohlenwasserstoffrest eine Cycloalkyl-alkylgruppe, so ist die Cyclopropylmethyl-, Cyclopropylethyl- und Cyclopentylmethylgruppe als bevorzugt anzusehen.

Geeignete durch ein Sauerstoffatom unterbrochene Cycloalkylgruppen sind beispielsweise 5-6 gliedrige cyclische Ethergruppen wie 3-Tetrahydrofuranyl und 3-Tetrahydropyranyl. Bedeutet der Kohlenwasserstoffrest eine Aryl- oder Aralkylgruppe so kann diese ein- bis dreifach substituiert sein, beispielsweise mit Halogen. Nitro,Cyano, Hydroxy, Mercapto, $C_{1-4}$-Alkyl, $C_{1-4}$-Alkoxy, $C_{1-4}$-Alkylthio, $C_{1-4}$-Alkylsulfinyl, $C_{1-4}$-Alkylsulfonyl oder einer gegebenenfalls mit $C_{1-4}$-Alkyl, $C_{1-4}$-Alkanoyl oder Sulfonyl- mono- oder disubstituierten Aminogruppe.

Als bevorzugter Arylrest sei Phenyl genannt, das gegebenenfalls ein- oder zweifach mit Halogen oder einer Cyano-, Nitro- oder gegebenenfalls substituierten Aminogruppe substituiert sein kann wie beispielsweise 2,4-Dichlorphenyl, 2-Cyanophenyl oder 4-Aminophenyl.

Der Aralkylrest $R^1$ kann im Alkylrest gerade oder verzweigt sein und im Arylrest gegebenenfalls ein- oder zweifach substituiert sein, vorzugsweise mit Halogen. $C_{1-4}$-Alkoxy, $C_{1-4}$-Alkyl oder mit gegebenenfalls substituiertem Amino.

Bevorzugt sind Ar-$C_{1-2}$-Alkyle, die im Arylrest durch 1 bis 2 Halogene insbesondere Brom und Chlor substituiert sein können wie beispielsweise Benzyl. Phenethyl, $\alpha$-Methylbenzyl, 4-Chlorphenethyl oder 3-Brombenzyl.

Bedeutet $R^1$ einen heteroaromatischen Rest, so kann dieser 5- oder 6-gliedrig sein und ein bis zwei Heteroatome wie Schwefel, Stickstoff und/oder Sauerstoff enthalten und gegebenenfalls substituiert sein mit den für den Arylrest genannten Substituenten.

Bevorzugt sind Sechsring-Heteroaromaten mit ein bis zwei Stickstoffatomen und Fünfringheteroaromaten mit ein bis zwei Sauerstoff-, Schwefel- und/oder Stickstoffatomen, die durch Halogen substituiert sein können, wie beispielsweise Pyridin, Pyrimidin, Pyrazin, Pyridazin, Furan, Thiophen, Pyrrol, Imidazol, Thiazol. Insbesondere seien als bevorzugte Reste Pyridin, Pyrimidin, Pyrazin, Thiazol und 5-Brompyridin genannt.

Bilden $R^1$ und $R^2$ gemeinsam mit dem Sauerstoffatom einen Ring, so kann die Kohlenwasserstoffbrücke 1-3 Kohlenstoffatome wie beispielsweise Methylen, Ethylen, Ethyliden, Propylen und außerdem ein weiteres Sauerstoffatom enthalten.

Bilden $R^6$, $R^7$ gemeinsam mit dem Stickstoffatom einen gegebenenfalls ein weiteres Heteroatom enthaltenden gesättigten heterocyclischen Fünf- oder Sechsring, so stellt dieser beispielsweise Pyrrolidin, Piperidin, Morpholin, Piperazin oder Thiomorpholin dar und kann gegebenenfalls mit ein bis zwei $C_{1-4}$-Alkylgruppen substituiert sein wie beispielsweise 2,6-Dimethylmorpholin oder N-Methylpiperazin.

Als bevorzugte Ausführungsform für $R^3$ in Bedeutung Alkoxyalkyl ist $C_{1-4}$-Alkoxy-$C_{1-4}$-Alkyl, insbesondere $C_{1-4}$-Alkoxymethyl anzusehen und für $R^4$ $COOR^5$,

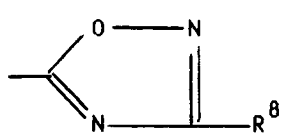

oder

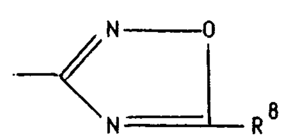

.

Die erfindungsgemäß substituierten Imidazolderivate zeigen, obwohl sie sich in ihrer chemischen Struktur von den Benzodiazepinen stark unterscheiden, überraschenderweise eine Affinität an die Benzodiazepin-Rezeptoren und weisen gleichzeitig nur geringe Toxizität auf. Benzodiazepine entfalten beispielsweise anticonvulsive, anxiolytische und muskelrelaxierende sowie sedierende Effekte. So können die erfindungsgemäßen Verbindungen beispielsweise auf die von Benzodiazepinen bekannten Eigenschaften agonistische, invers agonistische und antagonistische Effekte ausüben.

Die erfindungsgemäßen Verbindungen erscheinen aufgrund ihrer biologischen Wirksamkeit als Psychopharmaka, insbesondere als Anxiolytika, für die Humanmedizin geeignet. Sie können zu psychopharmazeutischen Präparationen, zum Beispiel für die orale und parenterale Anwendung, formuliert werden.

Als Formulierungshilfsstoffe sind physiologisch verträgliche organische und anorganische Trägersubstanzen geeignet, die gegenüber den erfindungsgemäßen Verbindungen inert sind.

Als Trägersubstanzen seien beispielsweise genannt Wasser, Salzlösungen, Alkohole, Polyäthylenglykole, polyhydroxyethoxyliertes Rizinusöl, Gelatine, Lactose , Amylose, Magnesiumstearat, Talkum, Kieselsäure, Fettsäure-mono- und -diglyceride, Pentaerythritolfettsäureester, Hydroxymethylcellulose und Polyvinylpyrrolidon.

Die pharmazeutischen Präparationen können sterilisiert und/oder mit Hilfsstoffen, wie Schmiermitteln, Konservierungsstoffen, Stabilisatoren, Netzmitteln, Emulgatoren, Puffermitteln und Farbstoffen, versetzt werden.

Für die parenterale Anwendung sind insbesondere Injektionslösungen oder Suspensionen, insbesondere wäßrige Lösungen der aktiven Verbindungen in polyhydroxyethoxyliertem Rizinusöl, geeignet. Als Trägersysteme können auch grenzflächenaktive Hilfsstoffe wie Salze der Gallensäuren oder tierische oder pflanzliche Phospholipide, aber auch Mischungen davon sowie Liposome oder deren Bestandteile verwendet werden.

Für die orale Anwendung sind insbesondere Tabletten, Dragees oder Kapseln mit Talkum und/oder einem Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt wird.

Die erfindungsgemäßen Verbindungen werden in einer Dosiseinheit von 0,05 bis 10 mg aktiver Substanz in einem physiologisch verträglichen Träger eingebracht.

Die erfindungsgemäßen Verbindungen werden in einer Dosis von 0,1 bis 300 mg/Tag vorzugsweise 1 - 30 mg/Tag, angewendet.

Die Herstellung der erfindungsgemäßen Verbindungen der allgemeinen Formel I erfolgt dadurch, daß man

a) ein Anilin der allgemeinen Formel II

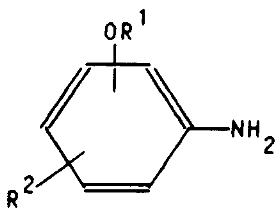

(II),

worin

$R^1$ und $R^2$ die in Formel I angegebene Bedeutung haben, mit einem 2-Azadien der allgemeinen Formel III

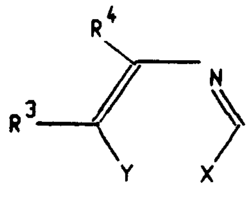

(III),

worin

$R^3$ und $R_4$ die in Formel I angegebene Bedeutung haben und

X und Y Fluchtgruppen darstellen,

in Gegenwart von Säuren bei Temperaturen von 0 bis 150 °C umsetzt oder

b) ein Imidazolderivat der allgemeinen Formel IV

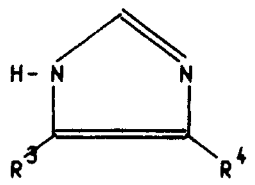

(IV),

worin
$R^3$ und $R^4$ die oben genannte Bedeutung haben, mit einem Aromaten der allgemeinen Formel V

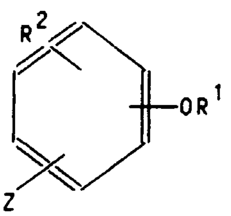

(V),

worin $R^1$ und $R^2$ die obige Bedeutung haben und Z eine Fluchtgruppe darstellt, aryliert und gegebenenfalls anschließend eine im Molekül vorhandene Estergruppe umestert oder verseift, eine freie Carboxylgruppe gegebenenfalls verestert, amidiert oder mit einem Amidoxim der Formel $R^8$-C(=NOH)$NH_2$ zum 5-Oxadiazolylderivat umsetzt und gegebenenfalls eine im Molekül vorhandene Nitrilgruppe zur Carbonylamid- oder Carboxygruppe hydrolisiert oder über die Iminoestergruppe in die Estergruppe ($COOR^5$) oder mit Hydroxylamin über das Amidoxim und anschließend mit einer Alkancarbonsäure der Formel $R^8$-COOH oder einem aktivierten Derivat der Säure in das 3-Oxadiazolylderivat überführt und gegebenenfalls Verbindungen mit $R^1$ = H in Gegenwart von Basen mit dem gegebenenfalls substituierten $C_{1-10}$-Kohlenwasserstoff- oder Hetarylrest verethert und gegebenenfalls eine Nitro-Gruppe zur Aminogruppe reduziert und diese anschließend gegebenenfalls alkyliert oder acyliert oder gegen Halogen, Azid, Cyano oder Thiocyanat austauscht.

Die erfindungsgemäße Umsetzung von Anilinen der Formel II mit 2-Azadienen der Formel III zu den Imidazolderivaten der Formel I erfolgt in Gegenwart von Säuren bei Temperaturen von 0 bis 150 °C. Die Fluchtgruppen X und Y können gleich oder verschieden sein; insbesondere geeignet sind $C_{1-3}$-Dialkylamine, wie Dimethyl-, Diethyl-und Dipropylamin, und cyclische Amine, wie Pyrrolidin.

Die Umsetzung wird beispielsweise so ausgeführt, daß das Anilinderivat und das Azadien in einer organischen Säure, wie zum Beispiel Ameisensäure, Essigsäure, Propionsäure oder Trifluoressigsäure, zunächst bei Raumtemperatur gerührt und dann bis zur Siedetemperatur des Reaktionsgemisches (bis etwa 120 °C) erhitzt wird.

Die Säure kann gleichzeitig als Reaktionsmittel und auch als Lösungsmittel dienen. Es können aber auch Lösungsmittel wie zum Beispiel Alkohole, Ether, Ketone, Ester, wie Ethylacetat, Kohlenwasserstoffe, wie Toluol, oder Halogenkohlenwasserstoffe, wie Tetrachlorkohlenstoff, zugesetzt werden.

Die Menge der Säure kann in weiten Grenzen variiert werden, sie wird jedoch im Überschuß angewendet. Vorzugsweise wird ein 3 - 10facher Säureüberschuß, bezogen auf das Anilin und das Azadien, gewählt.

Die Molverhältnisse von Anilin und Azadien sind für den Erfolg der Umsetzung nicht kritisch. Im allgemeinen wird man etwa gleiche molare Mengen der Reaktionspartner einsetzen, wobei Mengenverhältnisse von 1 Mol Anilin und 1-3 Mol Azadien bevorzugt sind. Die erfindungsgemäße Umsetzung kann grundsätzlich auch in den oben angegebenen Lösungsmitteln mit katalytischen Mengen von Mineralsäuren, wie Schwefelsäure, Salzsäure, Perchlorsäure oder organischen Säuren wie p-Toluolsulfonsäure und Trifluoressigsäure, durchgeführt werden.

Der Vorteil des erfindungsgemäßen Verfahrens nach der Methode a) liegt in der chemoselektiven Synthese von Imidazolderivaten unter Bildung nur eines Isomeren in einer einzigen Verfahrensstufe.

Die N-Arylierung der Imidazolderivate der allgemeinen Formel IV kann beispielsweise nach der von N.W. Gilman et al. J. Heterocycl. Chem. 14, 1157 (1977) beschriebenen Methode erfolgen. Hierbei ist es erforderlich, daß der Aromat der allgemeinen Formel V mit mindestens einer elektronenziehenden Gruppe

und mit einer Fluchtgruppe substituiert ist. Als elektronenziehende Gruppen sind insbesondere $NO_2$ und CN geeignet und als Fluchtgruppe Z kommen Halogene, insbesondere Fluor und Jod, in Betracht. Die Arylierung nach der Methode b) wird in Gegenwart von Basen wie Alkalihydroxid, Alkalihydrid gegebenenfalls in Gegenwart von Phasentransferkatalysatoren, Butyllithium oder Lithiumdiisopropylamid, vorzugsweise mit Alkalihydrid, durchgeführt.

Für die Umsetzung sind Temperaturen von - 78 °C bis 100 °C, vorzugsweise von 0 °C bis 50 °C geeignet.

Als Lösungsmittel für die Arylierung kommen aprotische polare Lösungsmittel in Betracht, beispielsweise aliphatische und cyclische Ether wie Diethylether oder Tetrahydrofuran und Dimethylformamid.

Für die sich gegebenenfalls anschließende Umesterung sind alle gebräuchlichen Methoden geeignet. Beispielsweise genannt seien die Umsetzung des Carbonsäureesters mit dem entsprechenden Alkohol in Gegenwart des Alkoholats oder mit dem entsprechenden Alkohol mit Titan-tetraalkoholat oder mit dem Alkohol in Gegenwart einer Säure . Die Umesterung wird bei Temperaturen von etwa 0 bis 120 °C durchgeführt.

Die sich gegebenenfalls anschließende Verseifung der Estergruppe erfolgt zweckmäßigerweise alkalisch, wobei der Ester in verdünnter wäßriger oder alkoholischer Alkalilauge, wie Kalium- oder Natriumhydroxid, bis zur Rückflußtemperatur erhitzt wird.

Die Veresterung der Carboxylgruppe geschieht in an sich bekannter Weise mit dem entsprechenden Alkohol in Säure oder in Gegenwart eines aktivierten Säurederivats. Als aktivierte Säurederivate kommen zum Beispiel Säurechlorid, -imidazolid oder -anhydrid infrage.

Zur Amidierung wird die Imidazol-4-carbonsäure oder der entsprechende Ester mit Hilfe von N,N`-Carbonyldiimidazol oder Dicyclohexylcarbodiimid mit einem primären oder sekundärem Amin der allgemeinen Formel

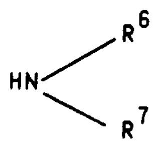

umgesetzt.

Die Umsetzung kann auch in an sich bekannter Weise über aktivierte Säurederivate wie über das Anhydrid oder das gemischte Anhydrid mit Chlorameisensäureester erfolgen. Üblicherweise wird die Amidierung in einem aprotischen Lösungsmittel wie Dimethylformamid, Tetrahydrofuran, Toluol oder Methylenchlorid bei Temperaturen von etwa 0 bis 100 °C vorgenommen.

Zur Einführung des 5-Oxadiazolylrestes kann auch die Imidazol-4-carbonsäure mit einem Amidoxim der Formel $R^8$-C(=NOH)$NH_2$, in der $R^8$ die in Formel I angegebene Bedeutung hat, in einem inerten Lösungsmittel bie Raumtemperatur bis zur Siedetemperatur des Reaktionsgemisches zur Kondensation gebracht werden. Als inerte Lösungsmittel sind beispielsweise Toluol und Dimethylformamid geeignet. Zweckmäßigerweise wird die freie Carbonsäure vor der Kondensationsreaktion in geeigneter Weise aktiviert. Hierzu kann die freie Säure in das gemischte Anhydrid, in den aktivierten Ester oder in das Chlorid überführt werden. Gut bewährt hat sich eine Aktivierung mit Imidazol/Thioylchlorid in einem aprotischen Lösungsmittel wie Dioxan, Tetrahydrofuran, Dimethylformamid oder N-Methylpyrrolidon bei Temperaturen zwischen 0 und 50 °C.

Die sich gegebenenfalls anschließende Abwandlung der Nitrilgruppe kann nach bekannten Methoden durchgeführt werden. Beispielsweise kann die Nitrilgruppe durch saure oder alkalische Hydrolyse in die Carbonylamid- oder Carboxylgruppe oder mit dem entsprechenden Alkohol unter Zugabe von Chlorwasserstoffgas über die Iminoestergruppe in die Estergruppe überführt werden.

Zur Einführung des 3-Oxadiazolylrestes wird das Imidazol-4-carbonitril in an sich bekannter Weise mit Hydroxylamin zum Amidoxim umgesetzt und anschließend mit einer Alkancarbonsäure der Formel $R^8$-COOH, in der $R^8$ die in Formel I angegebene Bedeutung hat, oder einem aktivierten Derivat der Säure in einem inerten Lösungsmittel kondensiert. Die Kondensation wird in der gleichen Weise wie bei der 5-Oxadiazolylverbindung durchgeführt.

Die Veretherung von Verbindungen der allgemeinen Formel I mit $R^1$ = H erfolgt nach an sich bekannten Methoden. Beispielsweise kann man ein reaktionsfähiges Derivat $R^1X$ in einem polaren Lösungsmittel in Gegenwart einer Base bei Temperaturen von Raumtemperatur bis zur Siedetemperatur des Lösungsmittels, gegebenenfalls auch in Gegenwart eines Phasentransferkatalysators, umsetzen. Als reaktionsfähiger Rest X ist insbesondere Halogen wie Chlor, Brom oder Jod sowie die Mesyl- oder Tosyl-Gruppe geeignet. Als Basen kommen Alkali-Verbindungen wie Natrium- oder Kalium-hydroxid, Natrium- oder Kalium-carbonat in Frage.

Die Reduktion der Nitrogruppe zur Aminogruppe kann beispielsweise katalytisch erfolgen, indem man unter Normaldruck oder $H_2$-Druck in polaren Lösungsmitteln bei Raumtemperatur hydriert. Als Katalysator kann Palladium auf einem Träger wie Kohle oder Platin in feinverteilter Form verwendet werden; bei Verbindungen mit Halogen verwendet man als Katalysator vorzugsweise Raney-Nickel. Für die Reduktion geeignete polare Lösungsmittel sind beispielsweise Alkohole oder Ether wie Methanol, Ethanol, Diethylether, Tetrahydrofuran oder deren Mischungen .

Die Einführung der Cyanogruppe kann mit Hilfe der Sandmeyer-Reaktion erfolgen; beispielsweise kann man die aus den Aminoverbindungen mit Nitriten intermediär gebildeten Diazoniumsalze mit Alkalicyaniden in Gegenwart von Cu-I-cyanid umsetzen.

Die Einführung der Halogene Chlor, Brom oder Jod über die Aminogruppe kann beispielsweise auch nach Sandmeyer erfolgen, indem man die mit Nitriten intermediär gebildete Diazoniumsalze mit Cu(I)chlorid oder Cu(I)bromid in Gegenwart der entsprechenden Säure Salzsäure oder Bromwasserstoffsäure umsetzt oder mit Kaliumiodid umsetzt.

Die Einführung von Fluor gelingt beispielsweise durch Balz Schiemann-Reaktion des Diazoniumtetrafluorborates.

Die Einführung der Azido- oder Thiocyanatgruppe kann ebenfalls über Sandmeyer-Reaktion des Diazoniumsalzes mit Alkaliazid oder Alkalithiocyanat erfolgen.

Wird eine Alkylierung oder Acylierung der Aminogruppe gewünscht, so kann nach üblichen Methoden beispielsweise mit Alkylhalogeniden oder Acylhalogeniden alkyliert oder acyliert werden.

Die als Ausgangsstoffe benutzten Aniline der allgemeinen Formel II und die Azadiene der allgemeinen Formel III sind überwiegend bekannt oder können nach bekannten Methoden hergestellt werden.

2-Azadiene sind zum Beispiel in Liebigs Ann. Chem. 1980, 344 und in DE-OS 29 19 891 und in Liebigs Ann. Chem. 1986, 1749 beschrieben.

Die Herstellung der in den Beispielen eingesetzten 2-Azadiene wird anhand der folgenden Beispiele erläutert:

**Azadien 1:**

1,4-Bis(dimethylamino)-2-aza-1,3-butadien-3-carbonsäure-ethylester:

Die Synthese erfolgt gemäß Liebigs Ann. Chem. 1980, 344.

**Azadien 2:**

1,4-Bis(dimethylamino)-3-(3-ethyl-1,2,4-oxadiazol-5-yl)-2-aza-1,3-butadien:

Die Synthese erfolgt gemäß Liebigs Ann. Chem. 1986, 1749.

**Azadien 3:**

1,4-Bis(dimethylamino)-3-cyano-2-azabutadien:

Eine Mischung aus 45 g Aminoacetonitril und 190 g Dimethylformamid-dimethylacetal wird unter Feuchtigkeitsausschluß 6 Stunden bei 150 °C (Badtemperatur) erhitzt. Dabei werden ca. 130 ml leichtflüchtiges Material (Methanol) abdestilliert. Nach fraktionierter Destillation im Vakuum erhält man 125,6 g (75,6 %) Azadien mit einem Siedepunkt von 115 - 125 °C / 0,04 mbar (0,03 Torr). Schmelzpunkt 78 - 81 °C (n-Hexan).

**Azadien 4:**

a) Dimethylaminomethylenacetonitril:

Führt man den in der Herstellungsvorschrift von Azadien 3 beschriebenen Ansatz bei niedrigerer Temperatur durch (Badtemperatur 100 °C), so erhält man 79,2 g (89 %) Dimethylaminomethylenacetonitril mit einem Siedepunkt von 64 - 67 °C / 0,13 mbar (0,1 Torr).

b) 3-Cyano-1-dimethylamino-4-pyrrolidino-2-aza-1,3-pentadien (E.Z-Gemisch):

Eine Mischung von 22g Dimethylaminomethylenacetonitril, 27 g Dimethylacetamiddimethylacetal und 14 g Pyrrolidin wird 48 Stunden auf 80 °C (Badtemperatur) erhitzt. Nach dem Einengen im Vakuum und anschließender Kugelrohrdestillation werden 30 g Azadien mit dem Siedepunkt 160 - 185 °C / 0,1 mbar (0,08 Torr) erhalten. Eine anschließende Kristallisation aus n-Hexan liefert Kristalle mit dem Schmelzpunkt 49 - 53 °C.

**Azadien 5:**

a) 3-Ethyl-5-(N-dimethylaminomethylenaminomethyl)-1,2,4-oxadiazol:

Eine Mischung aus 26 g 5-Aminomethyl-3-ethyl-1,2,4-oxadiazol und 30 ml Dimethylformamid-dimethylacetal wird 6 1/2 Stunden auf 80 °C (Badtemperatur) erhitzt. Anschließend werden 16 ml Methanol abdestilliert und das gebildete Produkt wird durch Kugelrohrdestillation gereinigt. Man erhält 27,9 g (74,8 %) Öl mit dem Siedepunkt 130 - 150 °C / 0,06 mbar (0,05 Torr); $n_D^{20}$: 1,4924.

b) 1-Dimethylamino-3-(3-ethyl-1,2-4-oxadiazol-5-yl)-4-pyrrolidinyl)-2-azapenta-1,3-dien (E.Z-Gemisch):

13,6 g des unter a erhaltenen Produktes, 15,0 g Dimethylacetamid-dimethylacetal und 8,0 g Pyrrolidin werden 21 Stunden unter Stickstoff auf 80 °C (Badtemperatur) erhitzt. Anschließend wird der gebildete Alkohol abdestilliert und das Reaktionsprodukt durch Kugelrohrdestillation gereinigt. Man erhält 15,4 g einer Fraktion, die bei 215 - 230 °C / 0,05 mbar (0,04 Torr) destilliert. Durch Umkristallisation aus n-Hexan gewinnt man 9,5 g (45,7 %) Azadien 5 mit einem Schmelzpunkt von 59 - 62 °C.

**Azadien 6:**

a) Methoxyessigsäure-dimethylamid-dimethylacetal:

Zu 53,4 g Trimethyloxonium-tetrafluoroborat werden unter Kühlung 42,2 g Methoxyessigsäure-dimethylamid in drei Portionen gegeben. Das Reaktionsgemisch wird anschließend 2 Stunden bei Raumtemperatur gerührt und danach über Nacht stehengelassen. Nach dem Lösen in 40 ml Dichlormethan wird das gebildete Salz langsam zu einer Lösung von Natriummethoxid in Methanol (hergestellt durch Auflösen von 10,4 g Natrium in 225 ml Methanol) gegeben. Danach wird noch 2 Stunden bei Raumtemperatur gerührt. Zur Aufarbeitung wird der gebildete Niederschlag abgesaugt und mit wenig Ethanol ausgewaschen. Das Filtrat bildet nach dem Abdestillieren der Lösungsmittel 2 Phasen. Durch Kugelrohrdestillation erhält man aus der oberen Phase 21,8 g (36 %) des gewünschten Produktes mit einem Siedepunkt von 54 - 57 °C / 18,7 mbar (14 Torr); $n_D^{20}$: 1,4204.

b) 1-Dimethylamino-3-(3-ethyl-1,2,4-oxadiazol-5-yl)-5-methoxy-4-(1-pyrrolidin yl)-2-azapenta-1,3-dien (E.Z-Gemisch)

8,8 g des unter a dargestellten Produktes werden mit 6,6 g 3-Ethyl-5-(N-dimethylaminomethylenaminomethyl)-1,2,4-oxadiazol und 4,5 ml Pyrrolidin analog der Herstellung von Azadien 5 umgesetzt. Durch Kugelrohrdestillation erhält man 11,8 g (quant.) Azadien 6 mit dem Siedepunkt 200 - 240 °C / 0,06 mbar (0,05 Torr).

**Beispiel 1**

a) 4-(3-Ethyl-1,2,4-oxadiazol-5-yl)-1-(3-hydroxyphenyl)-imidazol

Eine Lösung von 6,19 g Azadien 2 in 20 ml Eisessig wird unter Kühlung mit 2,18 g 3-Hydroxyanilin versetzt und anschließend 16 Stunden unter Stickstoff bei Raumtemperatur gerührt. Danach wird 1 1/2 Stunden auf 100 °C (Badtemperatur) erhitzt. Zur Aufarbeitung wird das Reaktionsgemisch unter Kühlung mit Natriumhydrogencarbonatlösung verdünnt, das gebildete Kristallisat abgesaugt, mit Wasser nachgewaschen und getrocknet. Durch Umkristallisation aus Ethanol erhält man 4,77 g (93,2 %) der Titelverbindung mit einem Schmelzpunkt von 214 -215 °C.

Analog Beispiel 1a) werden erhalten:

b) 4-(3-Ethyl-1,2,4-oxadiazol-5-yl)-1-(2-hydroxyphenyl)-imidazol

durch Umsetzung mit 2-Hydroxyanilin; Schmelzpunkt 200 - 202 °C (Isopropanol).

c) 1-(2,6-Dihydroxyphenyl)-4-(3-ethyl-1,2,4-oxadiazol-5-yl)-imidazol

durch Umsetzung mit 2,6-Dihydroxyanilin; Schmelzpunkt 239 - 240 °C (Ethanol).

**Beispiel 2**

1-(2,6-Dihydroxyphenyl)-imidazol-4-carbonsäure-ethylester

Eine Lösung von 2,8 g Azadien 1 in 10 ml Eisessig wird in der unter Beispiel 1a beschriebenen Weise mit 1,25 g 2,6-Dihydroxyanilin umgesetzt. Nach Aufarbeitung und Umkristallisation aus Acetonitril werden 1,9 g der Titelverbindung mit einem Schmelzpunkt von 229 - 230 °C erhalten.

**Beispiel 3**

a) 1-(2-Benzyloxyphenyl)-4-(3-ethyl-1,2,4-oxadiazol-5-yl)-imidazol

Ein Lösung von 256 mg der unter 1b hergestellten Verbindung in 2 ml wasserfreiem Dimethylformamid wird mit 83 mg Kaliumcarbonat versetzt und eine Stunde bei Raumtemperatur gerührt. Anschließend werden 152 mg Benzylchlorid zugegeben und das Reaktionsgemisch wird zunächst über Nacht bei Raumtemperatur, danach zur Vervollständigung der Reaktion eine Stunde bei 80 °C (Badtemperatur) geruht. Zur Aufarbeitung wird mit Ether verdünnt, der gebildete Niederschlag abgesaugt und getrocknet. Durch Umkristallisation aus Isopropanol werden 152 mg (43,9 %) der Titelverbindung mit einem Schmelzpunkt von 106 °C erhalten.

Analog werden erhalten:

b) 1-[2-(4-Chlorbenzyloxy)-phenyl]-4-(3-ethyl-1,2,4-oxadiazol-5-yl)-imidazol

durch Umsetzung der unter Beispiel 1b hergestgellten Verbindung mit 4-Chlorbenzylchlorid; Schmelzpunkt: 88 - 89 °C (Isopropanol).

c) 1-[2-(2-Phenylethoxy)-phenyl]-4-(3-ethyl-1,2,4-oxadiazol-5-yl)-imidazol

durch Umsetzung der unter Beispiel 1b hergestellten Verbindung mit 2-Phenethylbromid; Schmelzpunkt: 61 - 61 °C (Diisopropylether).

d) 1-(2,6-Dibenzyloxyphenyl)-4-(3-ethyl-1,2,4-oxadiazol-5-yl)-imidazol

durch Umsetzung der unter Beispiel 1c hergestellten Verbindung mit Benzylchlorid; Schmelzpunkt: 149 - 150 °C (Ethanol).

e) 1-(2,6-Dibenzyloxyphenyl)-imidazol-4-carbonsäure-ethylester

durch Umsetzung der unter Beispiel 2 hergestellten Verbindung mit Benzylchlorid; Schmelzpunkt: 125 - 126 °C (Isopropanol).

f) 4-(3-Ethyl-1,2,4-oxadiazol-5-yl)-1-[3-(2-phenyl-ethoxy)-phenyl]-imidazol

durch Umsetzung der unter Beispiel 1a hergestellten Verbindung mit 2-Phenethylbromid; Schmelzpunkt: 124 - 125 °C (Diisopropylether/Ethanol)

g) 1-[3-(4-Chlorbenzyloxy)-phenyl]-4-(3-ethyl-1,2,4-oxadiazol-5-yl)-imidazol

durch Umsetzung der unter Beispiel 1a hergestellten Verbindung mit 4-Chlorbenzylchlorid; Schmelzpunkt: 137 - 138 °C (Acetonitril)

**Beispiel 4**

4-(3-Ethyl-1,2,4-oxadiazol-5-yl)-1-[3-(4-nitrophenoxy)-phenyl]-imidazol

Eine Lösung von 2,56 g Azadien 2 in 40 ml wasserfreiem Dimethylformamid wird mit 976 mg Kaliumcarbonat versetzt und 15 Minuten unter Stickstoff bei 100 °C (Badtemperatur) gerührt. Nach dem Abkühlen auf Raumtemperatur gibt man 1,47 g 4-Fluor-nitrobenzol zu und rührt bis zur vollständigen Umsetzung bei 100 °C. Nach dem Abziehen des Lösungsmittels wird der Rückstand in Wasser aufgenommen, der gebildete Niederschlag abgesaugt und mit Wasser nachgewaschen. Durch Umkristallisation aus Ethanol erhält man 3,74 g (99,1 %) der Titelverbindung mit einem Schmelzpunkt von 145 - 146 °C.

**Beispiel 5**

1-[3-(4-Aminophenoxy)-phenyl]-4-(3-ethyl-1,2,4-oxadiazol-5-yl)-imidazol

745 mg der unter Beispiel 4 beschriebenen Verbindung werden in 80 ml Ethanol/Tetrahydrofuran (1 : 1) in Gegenwart von 3 g Raney-Nickel unter Normalbedingungen hydriert. Nach beendeter Wasserstoffaufnahme wird der Katalysator abgesaugt, gründlich nachgewaschen und das Filtrat eingeengt. Durch Chromatographie des Rohproduktes an Kieselgel mit Dichlormethan/Methanol (95 : 5) und anschließende Umkristallisation aus Diisopropylether erhält man 357 mg (51,5 %) der Titelverbindung mit einem Schmelzpunkt von 131 - 133 °C.

**Beispiel 6**

a) 1-(4-Benzyloxyphenyl)-4-(3-ethyl-1,2,4-oxadiazol-5-yl)-imidazol

Eine Lösung von 1,6 g Azadien 2 in 5 ml Eisessig wird in der unter Beispiel 1a beschriebenen Weise mit 1,0 g 4-Benzyloxyanilin umgesetzt. Nach Aufarbeitung und Umkristallisation aus Acetonitril werden 1,26 g (73 %) der Titelverbindung mit einem Schmelzpunkt von 171 - 172 °C erhalten.

Analog werden erhalten:

b) 1-(3-Benzyloxyphenyl)-4-(3-ethyl-1,2,4-oxadiazol-5-yl-imidazol

durch Umsetzung mit 3-Benzyloxyanilin; Schmelzpunkt: 113 - 114 °C (Ethanol).

c) 4-(3-Ethyl-1,2,4-oxadiazol-5-yl)-1-(3-phenoxyphenyl)-imidazol

durch Umsetzung mit 3-Phenoxyanilin; Schmelzpunkt: 108 - 109 °C (Acetonitril)

d) 1-[3-(4-Chlorphenoxy)-phenyl]-4-(3-ethyl-1,2,4-oxadiazol-5-yl)-imidazol

durch Umsetzung mit 3-(4-Chlorphenoxy)-anilin; Schmelzpunkt: 120 - 122 °C (Ethanol)

**Beispiel 7**

a) 1-(5-Chlor-2-methoxyphenyl-4-(3-ethyl-1,2,4-oxadiazol-5-yl)-imidazol

Eine Lösung von 619 mg Azadien 2 in 2 ml Eisessig wird in der unter Beispiel 1a beschriebenen Weise mit 315 mg 5-Chlor-2-methoxyanilin umgesetzt. Nach Aufarbeitung und Umkristallisation aus Ethanol werden 362 mg (59,4 %) der Titelverbindung mit dem Schmelzpunkt 139 - 140 °C erhalten.

Analog werden erhalten:

b) 1-(2-Chlor-5-methoxyphenyl)-4-(3-ethyl-1,2,4-oxadiazol-5-yl)-imidazol

durch Umsetzung mit 2-Chlor-5-methoxyanilin; Schmelzpunkt: 67 - 69 °C (Diisopropylether)

c) 1-(2-Benzyloxy-5-chlorphenyl)-4-(3-ethyl-1,2,4-oxadiazol-5-yl)-imidazol

durch Umsetzung mit 5-Chlor-2-benzyloxyanilin; Schmelzpunkt: 100 - 102 °C (Ethanol).

d) 1-(3,4-Methylendioxy-phenyl)-4-(3-ethyl-1,2,4-oxadiazol-5-yl)-imidazol

durch Umsetzung mit 3,4-Methylendioxyanilin; Schmelzpunkt: 173 - 174 °C (Ethanol).

e) 1-(3,4-Dimethoxyphenyl)-4-(3-ethyl-1,2,4-oxadiazol-5-yl)-imidazol

durch Umsetzung mit 3,4-Dimethoxyanilin; Schmelzpunkt: 143 °C (Ethanol).

f) 1-(3,4-Dimethoxyphenyl)-imidazol-4-carbonsäure-ethylester

durch Umsetzung von Azadien 1 mit 3,4-Dimethoxyanilin; Schmelzpunkt 125 - 126 °C (Ethylacetat).

**Beispiel 8**

a) 1-(3-Methoxyphenyl)-imidazol-4-carbonsäure-ethylester

Eine Lösung von 1,4 g Azadien 1 in 5 ml Eisessig wird mit 0,6 ml m-Anisidin versetzt und über Nacht bei Raumtemperatur gerührt. Zur Vervollständigung der Umsetzung wird danach noch eine Stunde bei 100 °C (Badtemperatur) gerührt. Nach üblicher Aufarbeitung und Chromatographie an Kieselgel mit Toluol/Ethanol ( 97 : 3 ) erhält man 1,18 g (96 %) der Titelverbindung mit dem Schmelzpunkt 95 - 96 °C (Toluol).

Analog werden erhalten:

b) 4-(3-Ethyl-1,2,4-oxadiazol-5-yl)-1-(3-methoxyphenyl)-imidazol

durch Umsetzung von Azadien 2 mit m-Anisidin; Schmelzpunkt: 89 - 90 °C (Ethanol).

c) 1-(3-Ethoxyphenyl)-4-(3-ethyl-1,2,4-oxyadiazol-5-yl)-imidazol

durch Umsetzung von Azadien 2 mit Phenetidin; Schmelzpunkt: 120 - 121 °C (Ethanol).

d) 1-(4-Methoxyphenyl)-imidazol-4-carbonsäure-ethylester

durch Umsetzung von Azadien 1 mit p-Anisidin; Schmelzpunkt: 93 - 94 °C (Toluol).

e) 4-(3-Ethyl-1,2,4-oxadiazol-5-yl)-1-(4-methoxyphenyl)-imidazol

durch Umsetzung von Azadien 2 mit p-Anisidin; Schmelzpunkt: 147 - 148 °C (Ethanol).

f) 1-(4-Phenoxyphenyl)-imidazol-4-carbonsäure-ethylester

durch Umsetzung von Azadien 1 mit p-Phenoxyanilin; Schmelzpunkt: 113 - 114 °C (Toluol).

g) 4-(3-Ethyl-1,2,4-oxadiazol-5-yl)-1-(4-phenoxyphenyl)-imidazol

durch Umsetzung von Azadien 2 mit p-Phenoxyanilin; Schmelzpunkt 172 - 173 °C (Ethanol).

**Beispiel 9**

1-(3-Methoxyphenyl)-imidazol-4-carbonsäure-isopropylester

Eine Lösung aus 615 mg der unter Beispiel 8a erhaltenen Verbindung und 0,8 ml Titanisopropylat wird unter Schutzgas 8 Stunden unter Rückfluß erhitzt. Anschließend wird das Reaktionsgemisch eingeengt, der Rückstand mit Essigester aufgenommen und mit wenig 2N HCl ausgeschüttelt. Danach wird mit Natriumcarbonat-Lösung neutralisiert, mit Essigester extrahiert, über Natriumsulfat getrocknet und eingeengt. Aus dem Rückstand erhält man durch Chromatographie an Kieselgel mit Hexan/Aceton (7 : 3) 560 mg

(86 %) der Titelverbindung als schwachgelbes Öl.

**Beispiel 10**

a) 4-(3-Ethyl-1,2,4-oxadiazol-5-yl)-1-[3-(2-propenyl)-oxyphenyl]-imidazol

Eine Lösung von 513 mg der unter Beispiel 1a hergestellten Verbindung in 10 ml wasserfreiem Dimethylformamid wird unter Stickstoff mit 165 mg Kaliumcarbonat versetzt und 15 Minuten auf 100 °C erhitzt. Nach dem Abkühlen werden 363 mg Allylbromid zugegeben und es wird erneut 3 1/2 Stunden auf 100 °C erhitzt. Danach wird das Lösungsmittel abdestilliert, der Rückstand mit Wasser verrieben, abgesaugt und gründlich mit Wasser nachgewaschen. Nach dem Trocknen wird das so gewonnene Rohprodukt durch Chromatographie an Kieselgel mit Toluol/Essigester (8 : 2) gereinigt. Nach dem Umkristallisieren aus Diisopropylether erhält man 454 mg (76,6 %) der Titelverbindung mit einem Schmelzpunkt von 73 - 74 °C.

Analog werden erhalten:

b) 4-(3-Ethyl-1,2,4-oxadiazol-5-yl)-1-(3-isopropyloxyphenyl)-imidazol

durch Umsetzung mit Isopropylbromid, Schmelzpunkt: 107 °C (Ethanol/n-Hexan).

c) 1-(3-Cyclopentyloxyphenyl)-4-(3-ethyl-1,2,4-oxadiazol-5-yl)-imidazol

durch Umsetzung mit Cyclopentylbromid; Schmelzpunkt: 132 °C (Diisopropylether).

d) [3-(5-Brom-2-pyridyloxy)-phenyl]-4-(3-ethyl-1,2,4-oxadiazol-5-yl)-imidazol

durch Umsetzung mit 2,5-Dibrompyridin; Schmelzpunkt: 141 - 142 °C (Ethanol).

**Beispiel 11**

1-(3-Hydroxyphenyl)-imidazol-4-carbonsäure-ethylester

13,91 g Azadien 1 und 5,46 g 3-Hydroxyanilin werden in 50 ml Eisessig analog Beispiel 1a umgesetzt. Nach Aufarbeitung des Reaktionsgemisches mit Natriumhydrogencarbonatlösung wird das erhaltene Rohprodukt aus Ethylacetat umkristallisiert. Man erhält auf diese Weise 11,17 g (96,2 %) der Titelverbindung mit einem Schmelzpunkt von 164 - 165 °C.

**Beispiel 12**

a) 1-(3-Isopropyloxyphenyl)-imidazol-4-carbonsäure-ethylester

Eine Mischung aus 696 mg der unter Beispiel 11 hergestellten Verbindung, 405 mg Isopropylbomid und 248 mg Kaliumcarbonat in 15 ml wasserfreiem Dimethylformamid wird unter Stickstoff eine Stunde auf 100 °C erhitzt. Bei unvollständiger Umsetzung gibt man noch zwei- bis dreimal äquivalente Mengen an Isopropylbromid und Kaliumcarbonat zu. Zur Aufarbeitung wird das Lösungsmittel abdestilliert und der verbleibende Rückstand mit Wasser verrührt. Das so erhaltene Rohprodukt wird abfiltriert, getrocknet und durch eine Chromatographie an Kieselgel mit Toluol/Essigester (8 : 2) aufgereinigt. Man erhält 782 mg (95 %) der Titelverbindung als schwachgelbes Öl.

Analog werden erhalten:

b) 1-(3-Cyclopentyloxyphenyl)-imidazol-4-carbonsäure-ethylester

durch Umsetzung mit Cyclopentylbromid; Schmelzpunkt: 66 - 67 °C (Cyclohexan).

c) 1-[3-(2-Propenyloxy)-phenyl]-imidazol-4-carbonsäure-ethylester

durch Umsetzung mit Allylbromid; Schmelzpunkt: 53 - 55 °C (Diisopropylether).

d) 1-[3-(5-Brom-2-pyridyloxy)-phenyl]-imidazol-4-carbonsäure-ethylester

durch Umsetzung mit 2,5-Dibrompyridin; Schmelzpunkt: 103 - 105 °C (Diisopropylether).

e) 1-[3-(4-Nitrophenoxy)-phenyl]-imidazol-4-carbonsäure-ethylester

durch Umsetzung mit 4-Fluor-nitrobenzol; Schmelzpunkt: 162 - 163 °C (Ethanol).

**Beispiel 13**

1-[3-(4-Aminophenoxy)-phenyl]-imidazol-4-carbonsäure-ethylester

2,83 g der unter Beispiel 12 e hergestellten Verbindung werden in 320 ml Ethanol/Tetrahydrofuran (2 : 1) in Gegenwart von 12 g Raney-Nickel unter Normalbedingungen hydriert. Nach vollendeter Wasserstoffaufnahme wird der Katalysator abfiltriert und gründlich mit Ethanol ausgewaschen. Aus dem Filtrat erhält man durch Einengen und Umkristallisation aus Ethanol 1,52 g (58,9 %) der Titelverbindung mit einem Schmelzpunkt von 162 - 163 °C.

**Beispiel 14**

1-[3-(4-Chlorphenoxy)-phenyl]-imidazol-4-carbonsäure-ethylester

1,3 g der unter Beispiel 13 hergestellten Verbindung werden bei - 5 °C in 30 ml conc. HCl/Wasser (1 : 1) gelöst und tropfenweise mit einer Lösung von 286 mg Natriumnitrit in 4 ml Wasser versetzt. Nachdem 50 Minuten nachgerührt wurde, gibt man das Reaktionsgemisch langsam zu einer gekühlten Lösung aus 455 mg Kupfer-(I)-chlorid in 6,7 ml conc. HCl. Es wird noch 15 Minuten bei - 5 - 0 °C nachgerührt. Anschließend wird das Gemisch langsam auf Raumtemperatur (ca. 30 Minuten) erwärmt, danach noch 30 Minuten bei 85 °C gerührt. Nach dem Abkühlen wird mit Wasser verdünnt und der entstandene Niederschlag abgesaugt. Dieser wird anschließend zwischen Essigester und einer 12 %igen Ammoniaklösung ausgeschüttelt. Die wäßrige Phase wird noch mehrmals gründlich mit Dichlormethan extrahiert. Anschließend werden die vereinigten organischen Phasen mit gesättigter Kochsalzlösung und Wasser gewaschen, über Natriumsulfat getrocknet und eingeengt. Aus dem Rohprodukt werden durch Chromatographie an Kieselgel mit einem Toluol/Essigester-Gradienten 312 mg (22,7 %) der Titelverbindung mit einem Schmelzpunkt von 112 - 113 °C (Ethanol) erhalten.

**Beispiel 15**

1-(3-Cyclopentyloxyphenyl)-imidazol-4-carbonsäure-isopropylester

480 mg der unter Beispiel 12 b hergestellten Verbindung werden in 20 ml wasserfreiem Isopropanol analog Beispiel 9 mit 277 mg Titanisopropylat umgesetzt. Nach Aufarbeitung und Chromatographie an Kieselgel mit Hexan/Aceton (8 : 2) erhält man 403 mg (80,1 %) der Titelverbindung als schwachgelbes Öl.

**Beispiel 16**

1-(3-Benzyloxyphenyl)-imidazol-4-carbonitril

Eine Lösung von 1,8 g Azadien 3 in 10 ml Eisessig wird mit 2g 3-Benzyloxyanilin versetzt und über Nacht bei Raumtemperatur gerührt. Anschließend wird das Reaktionsgemisch eine Stunde auf 100 °C erhitzt. Nach üblicher Aufarbeitung und Chromatographie an Kieselgel mit Toluol/Essigester (9 : 1) erhält man 2,4 g (86 %) der Titelverbindung als schwachgelbes Öl.

**Beispiel 17**

a) 1-(3-Hydroxyphenyl)-5-methyl-imidazol-4-carbonitril

Eine Lösung von 24,8 g Azadien 4 in 100 ml Eisessig wird mit 10,9 g 3-Hydroxyanilin versetzt und zwei Tage bei Raumtemperatur gerührt. Nachdem man anschließend noch fünf Stunden auf 100 °C erwärmt hat, wird das Reaktionsgemisch eingeengt, der Rückstand mit Essigester aufgenommen und mit Kaliumcarbonatlösung neutralisiert. Das nach dem Einengen der Essigester-Phase verbleibende Rohprodukt wird durch

Chromatographie an Kieselgel mit Dichlormethan/Methanol (95 : 5) und anschließende Umkristallisation aus Acetonitril aufgereinigt. Man erhält 6,64 g (33 %) der Titelverbindung mit einem Schmelzpunkt von 205-207 °C.

b) 1-(3-Hydroxyphenyl)-5-methyl-imidazol-4-formamidoxim

Eine Lösung von 6,37 g der unter 17a beschriebenen Verbindung in 250 ml wasserfreiem Ethanol wird mit 3,3 g Hydroxylammoniumchlorid und 6,6 g Kaliumcarbonat versetzt und drei Stunden refluxiert. Nach vollständiger Umsetzung wird das Lösungsmittel abdestilliert und der Rückstand zwischen Wasser und Essigester ausgeschüttelt. Nach dem Trocknen über Natriumsulfat und Einengen erhält man aus der Essigesterphase 6,6 g (88,9 %) der Titelverbindung, die ohne weitere Aufreinigung weiterverwendet wird.

c) 4-(5-Ethyl-1,2,4-oxadiazol-3-yl)-1-(3-hydroxy-phenyl)-5-methyl-imidazol

6,6 g des unter 17b beschriebenen Rohproduktes werden in 500 ml wasserfreiem Tetrahydrofuran gelöst und langsam mit 3,3 ml Propionylchlorid versetzt. Anschließend wird das Reaktionsgemisch über Nacht bei Raumtemperatur gerührt. Danach wird das Lösungsmittel abdestilliert, der Rückstand mit Xylol versetzt und 3 1/2 Stunden refluxiert. Nach dem Einengen wird das Rohprodukt durch Chromatographie an Kieselgel mit Dichlormethan/Methanol (95 : 5) aufgereinigt. Man erhält so 5,8 g (75 %) der Titelverbindung mit einem Schmelzpunkt von 226 - 227 °C (Acetonitril).

d) 1-(3-Cyclopentyloxyphenyl)-4-(5-ethyl-1,2,4-oxadiazol-3-yl)-5-methyl-imidazol

Eine Mischung aus 1,8 g der unter 17c hergestellten Verbindung, 730 mg Cyclopentylchlorid und 550 mg Kaliumcarbonat wird in 30 ml wasserfreiem Dimethylformamid eine Stunde unter Stickstoff auf 100 °C erhitzt. Zur Aufarbeitung wird das Lösungsmittel abdestilliert und der Rückstand mit Wasser verrührt. Das so erhaltene Rohprodukt wird getrocknet und durch eine Chromatographie an Kieselgel mit Toluol/Essigester (8 : 2) aufereinigt. Man erhält 1,63 g (73 %) der Titelverbindung als schwachgelbes Öl.

**Beispiel 18**

a) 5-Methyl-1-(3-phenoxyphenyl)-imidazol-4-carbonitril

Eine Lösung von 7,4 g Azadien 4 in 30 ml Eisessig wird mit 7,7 g 3-Phenoxyanilin versetzt und vier Tage bei Raumtemperatur gerührt. Danach wird das Reaktionsgemisch noch vier Stunden auf 100 °C erwärmt. Zur Aufarbeitung wird mit Kaliumcarbonatlösung neutralisiert und mit Essigester extrahiert. Die Essigesterphase wird mit Wasser gewaschen, über Natriumsulfat getrocknet und eingeengt. Das erhaltene Rohprodukt wird durch Chromatographie an Kieselgel mit Dichlormethan/Methanol (9 : 1) aufgereinigt. Nach Umkristallisation aus Methyltert.-butylether erhält man 2,44 g der Titelverbindung mit einem Schmelzpunkt von 97 - 98 °C.

b) 5-Methyl-1-(3-phenoxyphenyl)-imidazol-4-formamidoxim

2,2 g der unter 18 a beschriebenen Verbindung werden in 80 ml wasserfreiem Ethanol gelöst und mit 1,67 g Hydroxylammoniumchlorid und 1,66 g Kaliumcarbonat versetzt. Anschließend wird das Reaktionsgemisch zwei Stunden refluxiert. Zur Aufarbeitung wird das Lösungsmittel abdestilliert und der Rückstand mit Wasser verrührt. Die gebildeten Kristalle werden abgesaugt, getrocknet und aus Ethanol umkristallisiert. Man erhält 1,33 g (54 %) der Titelverbindung mit einem Schmelzpunkt von 196 - 198 °C.

c) 4-(5-Ethyl-1,2,4-oxadiazol-3-yl)-5-methyl-1-(3-phenoxyphenyl)-imidazol

Eine Lösung von 1,23 g der unter 18 b beschriebenen Verbindung in 100 ml wasserfreiem Tetrahydrofuran wird mit 0,47 ml Propionylchlorid versetzt und 16 Stunden bei Raumtemperatur gerührt. Nach dem Einengen wird der Rückstand in 40 ml Xylol aufgenommen und drei Stunden refluxiert. Zur Aufarbeitung wird das Lösungsmittel abdestilliert und das verbleibende Rohprodukt zwischen Kaliumcarbonatlösung und Essigester ausgeschüttelt. Aus der Essigesterphase erhält man nach dem Waschen mit Wasser und Trocknen über Natriumsulfat 1,15 g (83 %) der Titelverbindung als schwachgelbes Öl.

**Beispiel 19**

1-(3-Benzyloxyphenyl)-4-(3-ethyl-1,2,4-oxadiazol-5-yl)-5-methyl-imidazol

Eine Lösung von 1 g Azadien 5 in 5 ml Eisessig wird mit 0,58 g 3-Benzyloxyanilin versetzt und 16 Stunden bei Raumtemperatur gerührt. Anschließend wird das Reaktionsgemisch eine Stunde auf 100 °C erwärmt. Nach Aufarbeitung und Chromatographie an Kieselgel mit Toluol/Ethanol (9 : 1) erhält man 682 mg (65%) der Titelverbindung als schwachgelbes Öl.

**Beispiel 20**

a) 4-(3-Ethyl-1,2,4-oxadiazol-5-yl)-1-(3-hydroxyphenyl)-5-methoxymethyl-imidazol

Eine Lösung von 15,4 g Azadien 6 in 42 ml Eisessig wird mit 4,55 g 3-Hydroxyanilin versetzt und drei Tage bei Raumtemperatur unter Stickstoff gerührt. Anschließend wird noch zwei Stunden auf 100 °C erwärmt. Zur Aufarbeitung wird das Reaktionsgemisch mit Wasser verdünnt, mit Kaliumcarbonatlösung alkalisch gestellt und mit Essigester extrahiert. Die Essigester-Phase wird nacheinander mit Kaliumcarbonatlösung, Kochsalzlösung und Wasser gewaschen, über Natriumsulfat getrocknet und eingeengt. Aus dem Rohprodukt gewinnt man durch Chromatographie an Kieselgel mit einem Toluol/Essigester-Gradienten 9,24 g (73,8 %) der Titelverbindung mit einem Schmelzpunkt von 98 - 99 °C (Methyl-tert.-butylether).

Analog werden erhalten:

b) 4-(3-Ethyl-1-,2,4-oxadiazol-5-yl)-5-methoxymethyl-1-(3-methoxyphenyl)-imidazol

durch Umsetzung mit 3-Methoxyanilin; Schmelzpunkt: 66 - 68 °C (n-Hexan)

c) 1-[3-(4-Chlorphenoxy)-phenyl]-4-(3-ethyl-1,2,4-oxadiazol-5-yl)-5-methoxymethyl-imidazol

durch Umsetzung mit 3-(4-Chlorphenoxy)-anilin; Schmelzpunkt: 68 - 70 °C (Cyclohexan/n-Hexan)

d) 1-(3-Benzyloxyphenyl)-4-(3-ethyl-1,2,4-oxadiazol-5-yl)-5-methoxymethyl-imidazol

durch Umsetzung mit 3-Benzyloxyanilin; Schmelzpunkt: 63 - 64 °C (n-Hexan)

**Beispiel 21**

a) 1-(3-Cyclopentyloxyphenyl)-4-(3-ethyl-1,2,4-oxadiazol-5-yl)-5-methoxymethyl-imidazol

Eine Lösung von 901 mg der unter Beispiel 20 a beschriebenen Verbindung in 15 ml wasserfreiem Dimethylformamid wird mit 980 mg Cyclopentylbromid und 500 mg Kaliumcarbonat versetzt und sechs Stunden auf 100 °C erwärmt. Zur Aufarbeitung wird das Lösungsmittel abdestilliert, der Rückstand mit Wasser versetzt und mit Essigester extrahiert. Die Essigester-Phase wird mit Wasser gewaschen, über Natriumsulfat getrocknet und eingeengt. Durch Chromatographie an Kieselgel mit Toluol/Essigester (9 : 1) gewinnt man 1,07 g (96,8 %) der Titel verbindung als schwachgelbes Öl.

Analog werden erhalten:

b) 4-(3-Ethyl-1,2,4-oxadiazol-5-yl)-5-methoxymethyl-1-[3-(4-nitrophenoxy)-phenyl]-imidazol

durch Umsetzung mit 4-Fluornitrobenzol; Schmelzpunkt 126 - 127 °C (Acetonitril)

c) 1-[3-(4-Chlorbenzyloxy)-phenyl]-4-(3-ethyl-1,2,4-oxadiazol-5-yl)-5-methoxy methyl-imidazol

durch Umsetzung mit 4-Chlorbenzylchlorid; schwachgelbes Öl

**Patentansprüche**

**1.** Imidazolderivate der allgemeinen Formel I

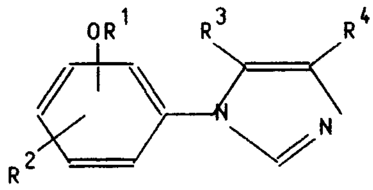

(I)

worin

$R^1$ Wasserstoff, einen gegebenenfalls ein- bis dreifach mit Halogen, Nitro, Cyano, Hydroxy, Mercapto, $C_{1-4}$-Alkyl, $C_{1-4}$-Alkoxy, $C_{1-4}$-Alkylthtio, $C_{1-4}$-Alkylsulfinyl, $C_{1-4}$-Alkylsulfonyl oder einer gegebenenfalls mit $C_{1-4}$-Alkyl, $C_{1-4}$-Alkanoyl oder Sulfonyl mono- oder disubstituierten Aminogruppe substituierten $C_{1-10}$-Kohlenwasserstoff- oder 5-6 gliedrigen Hetarylrest, der ein bis zwei Schwefel-, Stickstoff- und/oder Sauerstoffatome enthält oder einen 5-6-gliedrigen cyclischen Etherrest bedeutet,

$R^2$ Wasserstoff, Halogen, eine gegebenenfalls mit $C_{1-4}$-Alkyl, $C_{1-4}$-Alkanoyl oder Sulfonyl mono- oder disubstituierte Aminogruppe, eine Nitro-, Azid-, Thiocyanat- oder Cyanogruppe, einen gegebenenfalls mit Halogen substituierten geraden oder verzweigten $C_{1-10}$-Alkylrest oder -$OR^1$ mit $R^1$ in der vorne genannten Bedeutung und

$R^1$ und $R^2$ gemeinsam mit dem Sauerstoffatom einen gesättigten oder ungesättigten 5- bis 7-gliedrigen Ring bilden, der ein weiteres Sauerstoffatom enthalten kann,

$R^3$ Wasserstoff, eine gerade oder verzweigte $C_{1-6}$-Alkylgruppe oder eine $C_{1-6}$-Alkoxyalkylgruppe,

$R^4$ -$COOR^5$, -$CONR^6R^7$, -CN,

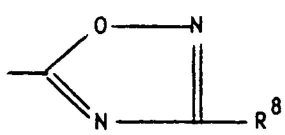

oder

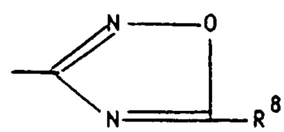

mit $R^5$ in der Bedeutung von Wasserstoff, einer geraden oder verzweigten $C_{1-6}$-Alkylgruppe, $R^6$ und $R^7$ sind gleich oder verschieden und stellen Wasserstoff oder eine gerade, verzweigte oder cyclische Alkylgruppe mit bis zu 7 Kohlenstoffatomen dar oder bilden gemeinsam mit dem Stickstoffatom gegebenenfalls mit ein bis zwei $C_{1-4}$-Alkyl substituiertes Morpholin, Piperazin, Pyrrolidin, Piperidin oder Thiomorpholin und $R^8$ in der Bedeutung von Wasserstoff oder einer geraden, verzweigten oder cyclischen Alkylgruppe mit bis zu 7 Kohlenstoffatomen,

wobei $R^2$ ein- oder mehrfach am Phenylrest stehen kann.

**2.** 4-(3-Ethyl-1,2,4-oxadiazol-5-yl)-1-(2-hydroxyphenyl)-imidazol
1-(2-Benzyloxyphenyl)-4-(3-ethyl-1,2,4-oxadiazol-5-yl)-imidazol
1-[2-(4-Chlorbenzyloxy)-phenyl]-4-(3-ethyl-1,2,4-oxadiazol-5-yl)-imidazol
1-[2-(2-Phenylethoxy)-phenyl]-4-(3-ethyl-1,2,4-oxadiazol-5-yl)-imidazol
4-(3-Ethyl-1,2,4-oxadiazol-5-yl)-1-(3-hydroxyphenyl)-imidazol
1-(3-Benzyloxyphenyl)-4-(3-ethyl-1,2,4-oxadiazol-5-yl)-imidazol
1-[3-(4-Chlorbenzyloxy)-phenyl]-4-(3-ethyl-1,2,4-oxadiazol-5-yl)-imidazol
4-(3-Ethyl-1,2,4-oxadiazol-5-yl)-1-[3-(2-phenylethoxy)-phenyl]-imidazol
4-(3-Ethyl-1,2,4-oxadiazol-5-yl)-1-[3-(4-nitrophenoxy)phenyl]-imidazol
1-[3-(4-Aminophenoxy)-phenyl]-4-(3-ethyl-1,2,4-oxadiazol-5-yl)-imidazol
1- (4-Benzyloxyphenyl)-4-(3-ethyl-1,2,4-oxadiazol-5-yl)-imidazol
4-(3-Ethyl-1,2,4-oxadiazol-5-yl)-1-(3-phenoxyphenyl)-imidazol
1-[3-(4-Chlorphenoxy)-phenyl]-4-(3-ethyl-1,2,4-oxadiazol-5-yl)-imidazol
1-(5-Chlor-2-methoxyphenyl)-4-(3-ethyl-1,2,4-oxadiazol-5-yl)-imidazol
1-(2-Benzyloxy-5-chlorphenyl)-4-(3-ethyl-1,2,4-oxadiazol-5-yl)-imidazol
1-(2-Chlor-5-methoxyphenyl)-4-(3-ethyl-1,2,4-oxadiazol-5-yl)-imidazol

1-(3,4-Methylendioxy-phenyl)-4-(3-ethyl-1,2,4-oxadiazol-5-yl)-imidazol
1-(3,4-Dimethoxyphenyl)-imidazol-4-carbonsäureethylester
1-(3,4-Dimethoxyphenyl)-4-(3-ethyl-1,2,4-oxadiazol-5-yl)-imidazol
1-(2,6-Dihydroxyphenyl)-4-(3-ethyl-1,2,4-oxadiazol-5-yl)-imidazol
1-(2,6-Dihydroxyphenyl)-imidazol-4-carbonsäureethylester
1-(2,6-Dibenzyloxyphenyl)-imidazol-4-carbonsäureethylester
1-(2,6-Dibenzyloxyphenyl)-4-(3-ethyl-1,2,4-oxadiazol-5-yl)-imidazol
1-(3-Methoxyphenyl)-imidazol-4-carbonsäureethylester
1-(3-Methoxyphenyl)-imidazol-4-carbonsäureisopropylester
4-(3-Ethyl-1,2,4-oxadiazol-5-yl)-1-(3-methoxyphenyl)-imidazol
1-(3-Ethoxyphenyl)-4-(3-ethyl-1,2,4-oxadiazol-5-yl)-imidazol
1-(4-Methoxyphenyl)-imidazol-4-carbonsäureethylester
4-(3-Ethyl-1,2,4-oxadiazol-5-yl)-1-(4-methoxyphenyl)-imidazol
1-(4-Phenoxyphenyl)-imidazol-4-carbonsäureethylester
4-(3-Ethyl-1,2,4-oxadiazol-5-yl)-1-(4-phenoxyphenyl)-imidazol
4-(3-Ethyl-1,2,4-oxadiazol-5-yl)-1-[3-(2-propenyl)-oxyphenyl]-imidazol
4-(3-Ethyl-1,2,4-oxadiazol-5-yl)-1-(3-isopropyl-oxyphenyl)-imidazol
1-(3-Cyclopentyloxyphenyl)-4-(3-ethyl-1,2,4-oxadiazol-5-yl)-imidazol
[3-(5-Brom-2-pyridyloxy)-phenyl]-4-(3-ethyl-1,2,4-oxadiazol-5-yl)-imidazol
1-(3-Hydroxyphenyl)-imidazol-4-carbonsäure-ethylester
1-(3-Isopropyloxyphenyl)-imidazol-4-carbonsäure-ethylester
1-(3-Cyclopentyloxyphenyl)-imidazol-4-carbonsäure-ethylester
1-[3-(2-Propenyloxy)-phenyl]-imidazol-4-carbonsäure-ethylester
1-[3-(5-Brom-2-pyridyloxy)-phenyl]-imidazol-4-carbonsäure-ethylester
1-[3-(4-Nitrophenoxy)-phenyl]-imidazol-4-carbonsäure-ethylester
1-[3-(4-Aminophenoxy)-phenyl]-imidazol-4-carbonsäure-ethylester
1-[3-(4-Chlorphenoxy)-phenyl]-imidazol-4-carbonsäure-ethylester
1-(3-Cyclopentyloxyphenyl)-imidazol-4-carbonsäure-isopropylester
1-(3-Benzyloxyphenyl)-imidazol-4-carbonitril
1-(3-Hydroxyphenyl)-5-methyl-imidazol-4-carbonitril
4-(5-Ethyl-1,2,4-oxadiazol-3-yl)-1-(3-hydroxyphenyl)-5-methyl-imidazol
1-(3-Cyclopentyloxyphenyl)-4-(5-ethyl-1,2,4-oxadiazol-3-yl-)-5-methyl-imidazol
5-Methyl-1-(3-phenoxyphenyl)-imidazol-4-carbonitril
4-(5-Ethyl-1,2,4-oxadiazol-3-yl)-5-methyl-1-(3-phenoxyphenyl)-imidazol
1-(3-Benzyloxyphenyl)-4-(3-ethyl-1,2,4-oxadiazol-5-yl)-5-methyl-imidazol
4-(3-Ethyl-1,2,4-oxadiazol-5-yl)-1-(3-hydroxyphenyl)-5-methoxymethyl-imidazol
4-(3-Ethyl-1,2,4-oxadiazol-5-yl)-5-methoxymethyl-1(3-methoxyphenyl)-imidazol
1-[3(4-Chlorphenoxy)-phenyl]-4-(3-ethyl-1,2,4-oxadiazol-5-yl)-5-methoxy-methyl-imidazol
1-(3-Benzyloxyphenyl)-4-(3-ethyl-1,2,4-oxadiazol-5-yl)-5-methoxymethyl-imidazol
1-(3-Cyclopentyloxyphenyl)-4-(3-ethyl-1,2,4-oxadiazol-5-yl)-5-methoxy-methyl-imidazol
4-(3-Ethyl-1,2,4-oxadizol-5-yl)-5-methoxymethyl-1-[3-(4-nitrophenoxy)-phenyl]-imidazol
1-[3-(4-Chlorbenzyloxy)-phenyl]-4-(3-ethyl-1,2,4-oxadiazol-5-yl)-5-methoxymethyl-imidazol
nach Anspruch 1.

**3.** Imidazolderivate der allgemeinen Formel I gemäß Anspruch 1, worin $R^4$ $COOR^5$`

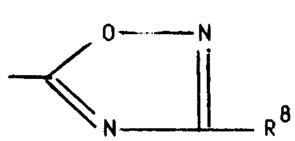

oder

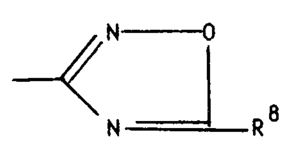

ist.

**4.** Arzneimittel auf Basis der Verbindungen gemäß Anspruch 1-3.

**5.** Verfahren zur Herstellung der Verbindungen der Formel I gemäß Anspruch 1, dadurch gekennzeichnet, daß man

a) ein Anilin der allgemeinen Formel II

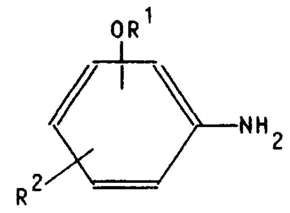

(II),

worin
$R^1$ und $R^2$ die in Formel I angegebene Bedeutung haben, mit einem 2-Azadien der allgemeinen Formel III

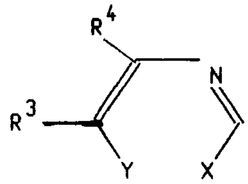

(III),

worin
$R^3$ und $R^4$ die in Formel I angegebene Bedeutung haben und
X und Y Fluchtgruppen darstellen,
in Gegenwart von Säuren bei Temperaturen von 0 bis 150 °C umsetzt oder
b) ein Imidazolderivat der allgemeinen Formel IV

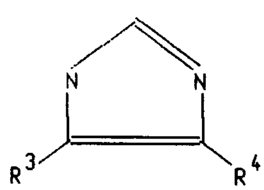

(IV),

worin
$R^3$ und $R^4$ die oben genannte Bedeutung haben, mit einem Aromaten der allgemeinen Formel V

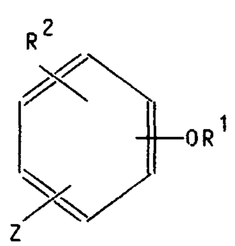

(V),

worin
$R^1$ und $R^2$ die obige Bedeutung haben und Z eine Fluchtgruppe darstellt, aryliert, und gegebenenfalls anschließend in den nach a) oder b) erhaltenen Verbindungen eine im Molekül vorhandene Estergruppe umestert oder verseift, eine freie Carboxylgruppe gegebenenfalls verestert, amidiert oder mit einem Amidoxim der Formel $R^8$-C(=NOH)$NH_2$ zum 5-Oxadiazolylderivat umsetzt und gegebenenfalls eine im Molekül vorhandene Nitrilgruppe zur Carbonylamid- oder Carboxylgruppe hydrolisiert oder über die Iminoestergruppe in die Estergruppe ($COOR^5$) oder mit Hydroxylamin über

das Amidoxim und anschließend mit einer Alkancarbonsäure der Formel $R^8$-COOH oder einem aktivierten Derivat der Säure in das 3-Oxadiazolylderivat überführt und gegebenenfalls Verbindungen mit $R^1$ = H in Gegenwart von Basen mit dem gegebenenfalls substituierten $C_{1-10}$-Kohlenwasserstoff-oder Hetarylrest verethert und gegebenenfalls eine Nitro-Gruppe zur Aminogruppe reduziert und diese anschließend gegebenenfalls alkyliert oder acyliert oder gegen Halogen, Azid, Cyano oder Thiocyanat austauscht.

**6.** Verfahren zur Herstellung eines Arzneimittels dadurch, daß man eine nach Anspruch 5 hergestellte Verbindung der Formel I mit einem pharmazeutischen Träger mischt.

## Claims

**1.** Imidazole derivatives of the general formula I

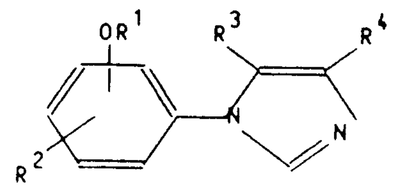

(I)

wherein

$R^1$ represents hydrogen, a $C_{1-10}$hydrocarbon or 5- to 6-membered hetaryl radical having one or two sulphur, nitrogen and/or oxygen atoms, each of which is optionally mono- to tri-substituted by halogen, nitro, cyano, hydroxy, mercapto, $C_{1-4}$alkyl, $C_{1-4}$alkoxy, $C_{1-4}$alkylthio, $C_{1-4}$alkylsulphinyl, $C_{1-4}$alkylsulphonyl or by an amino group optionally mono- or di-substituted by $C_{1-4}$alkyl, $C_{1-4}$alkanoyl or by sulphonyl, or represents a 5- to 6-membered cyclic ether radical,

$R^2$ represents hydrogen, halogen, an amino group optionally mono- or di-substituted by $C_{1-4}$alkyl, $C_{1-4}$alkanoyl or by sulphonyl, a nitro, azide, thiocyanate or cyano group, an optionally halo-substituted linear or branched $C_{1-10}$alkyl radical, or -$OR^1$ wherein $R^1$ has the meaning given above, and

$R^1$ and $R^2$ jointly with the oxygen atom form a saturated or unsaturated 5- to 7-membered ring which may contain a further oxygen atom,

$R^3$ represents hydrogen, a linear or branched $C_{1-6}$alkyl group or a $C_{1-6}$alkoxyalkyl group,

$R^4$ represents -$COOR^5$, -$CONR^6R^7$, -CN,

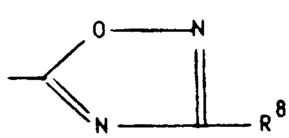

or

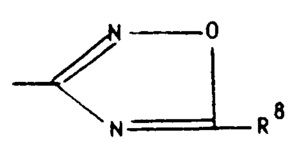

in which $R^5$ represents hydrogen, a linear or branched $C_{1-6}$alkyl group, $R^6$ and $R^7$ are identical or different and represent hydrogen or a linear, branched or cyclic alkyl group having up to 7 carbon atoms or form jointly with the nitrogen atom optionally $C_{1-4}$alkyl-mono- or di-substituted morpholine, piperazine, pyrrolidine, piperidine or thiomorpholine, and $R^8$ represents hydrogen or a linear, branched or cyclic alkyl group having up to 7 carbon atoms,

there being one or more radicals $R^2$ present at the phenyl radical.

**2.** 4-(3-ethyl-1,2,4-oxadiazol-5-yl)-1-(2-hydroxyphenyl)-imidazole,
1-(2-benzyloxyphenyl)-4-(3-ethyl-1,2,4-oxadiazol-5-yl)-imidazole,
1-[2-(4-chlorobenzyloxy)-phenyl]-4-(3-ethyl-1,2,4-oxadiazol-5-yl)-imidazole,
1-[2-(2-phenylethoxy)-phenyl]-4-(3-ethyl-1,2,4-oxadiazol-5-yl)-imidazole,

4-(3-ethyl-1,2,4-oxadiazol-5-yl)-1-(3-hydroxyphenyl)-imidazole,
1-(3-benzyloxyphenyl)-4-(3-ethyl-1,2,4-oxadiazol-5-yl)-imidazole,
1-[3-(4-chlorobenzyloxy)-phenyl]-4-(3-ethyl-1,2,4-oxadiazol-5-yl)-imidazole,
4-(3-ethyl-1,2,4-oxadiazol-5-yl)-1-[3-(2-phenylethoxy)-phenyl]-imidazole,
4-(3-ethyl-1,2,4-oxadiazol-5-yl)-1-[3-(4-nitrophenoxy)-phenyl]-imidazole,
1-[3-(4-aminophenoxy)-phenyl]-4-(3-ethyl-1,2,4-oxadiazol-5-yl)-imidazole,
1-(4-benzyloxyphenyl)-4-(3-ethyl-1,2,4-oxadiazol-5-yl)-imidazole,
4-(3-ethyl-1,2,4-oxadiazol-5-yl)-1-(3-phenoxyphenyl)-imidazole,
1-[3-(4-chlorophenoxy)-phenyl]-4-(3-ethyl-1,2,4-oxadiazol-5-yl)-imidazole,
1-(5-chloro-2-methoxyphenyl)-4-(3-ethyl-1,2,4-oxadiazol-5-yl)-imidazole,
1-(2-benzyloxy-5-chlorophenyl)-4-(3-ethyl-1,2,4-oxadiazol-5-yl)-imidazole,
1-(2-chloro-5-methoxyphenyl)-4-(3-ethyl-1,2,4-oxadiazol-5-yl)-imidazole,
1-(3,4-methylenedioxyphenyl)-4-(3-ethyl-1,2,4-oxadiazol-5-yl)-imidazole,
1-(3,4-dimethoxyphenyl)-imidazole-4-carboxylic acid ethyl ester,
1-(3,4-dimethoxyphenyl)-4-(3-ethyl-1,2,4-oxadiazol-5-yl)-imidazole,
1-(2,6-dihydroxyphenyl)-4-(3-ethyl-1,2,4-oxadiazol-5-yl)-imidazole,
1-(2,6-dihydroxyphenyl)-imidazole-4-carboxylic acid ethyl ester,
1-(2,6-dibenzyloxyphenyl)-imidazole-4-carboxylic acid ethyl ester,
1-(2,6-dibenzyloxyphenyl)-4-(3-ethyl-1,2,4-oxadiazol-5-yl)-imidazole,
1-(3-methoxyphenyl)-imidazole-4-carboxylic acid ethyl ester,
1-(3-methoxyphenyl)-imidazole-4-carboxylic acid isopropyl ester,
4-(3-ethyl-1,2,4-oxadiazol-5-yl)-1-(3-methoxyphenyl)-imidazole,
1-(3-ethoxyphenyl)-4-(3-ethyl-1,2,4-oxadiazol-5-yl)-imidazole,
1-(4-methoxyphenyl)-imidazole-4-carboxylic acid ethyl ester,
4-(3-ethyl-1,2,4-oxadiazol-5-yl)-1-(4-methoxyphenyl)-imidazole,
1-(4-phenoxyphenyl)-imidazole-4-carboxylic acid ethyl ester,
4-(3-ethyl-1,2,4-oxadiazol-5-yl)-1-(4-phenoxyphenyl)-imidazole,
4-(3-ethyl-1,2,4-oxadiazol-5-yl)-1-[3-(2-propenyl)-oxyphenyl]-imidazole,
4-(3-ethyl-1,2,4-oxadiazol-5-yl)-1-(3-isopropyloxyphenyl)-imidazole,
1-(3-cyclopentyloxyphenyl)-4-(3-ethyl-1,2,4-oxadiazol-5-yl)-imidazole,
[3-(5-bromo-2-pyridyloxy)-phenyl]-4-(3-ethyl-1,2,4-oxadiazol-5-yl)-imidazole,
1-(3-hydroxyphenyl)-imidazole-4-carboxylic acid ethyl ester,
1-(3-isopropoxyphenyl)-imidazole-4-carboxylic acid ethyl ester,
1-(3-cyclopentyloxyphenyl)-imidazole-4-carboxylic acid ethyl ester,
1-[3-(2-propenyloxy)-phenyl]-imidazole-4-carboxylic acid ethyl ester,
1-[3-(5-bromo-2-pyridyloxy)-phenyl]-imidazole-4-carboxylic acid ethyl ester,
1-[3-(4-nitrophenoxy)-phenyl]-imidazole-4-carboxylic acid ethyl ester,
1-[3-(4-aminophenoxy)-phenyl]-imidazole-4-carboxylic acid ethyl ester,
1-[3-(4-chlorophenoxy)-phenyl]-imidazole-4-carboxylic acid ethyl ester,
1-(3-cyclopentyloxyphenyl)-imidazole-4-carboxylic acid isopropyl ester,
1-(3-benzyloxyphenyl)-imidazole-4-carbonitrile,
1-(3-hydroxyphenyl)-5-methylimidazole-4-carbonitrile,
4-(5-ethyl-1,2,4-oxadiazol-3-yl)-1-(3-hydroxyphenyl)-5-methylimidazole,
1-(3-cyclopentyloxyphenyl)-4-(5-ethyl-1,2,4-oxadiazol-3-yl)-5-methylimidazole,
5-methyl-1-(3-phenoxyphenyl)-imidazole-4-carbonitrile,
4-(5-ethyl-1,2,4-oxadiazol-3-yl)-5-methyl-1-(3-phenoxyphenyl)-imidazole,
1-(3-benzyloxyphenyl)-4-(3-ethyl-1,2,4-oxadiazol-5-yl)-5-methylimidazole,
4-(3-ethyl-1,2,4-oxadiazol-5-yl)-1-(3-hydroxyphenyl)-5-methoxymethylimidazole,
4-(3-ethyl-1,2,4-oxadiazol-5-yl)-5-methoxymethyl-1-(3-methoxyphenyl)-imidazole,
1-[3-(4-chlorophenoxy)-phenyl]-4-(3-ethyl-1,2,4-oxadiazol-5-yl)-5-methoxymethylimidazole,
1-(3-benzyloxyphenyl)-4-(3-ethyl-1,2,4-oxadiazol-5-yl)-5-methoxymethylimidazole,
1-(3-cyclopentyloxyphenyl)-4-(3-ethyl-1,2,4-oxadiazol-5-yl)-5-methoxymethylimidazole,
4-(3-ethyl-1,2,4-oxadiazol-5-yl)-5-methoxymethyl-1-[3-(4-nitrophenoxy)-phenyl]-imidazole,
1-[3-(4-chlorobenzyloxy)-phenyl)-4-(3-ethyl-1,2,4-oxadiazol-5-yl)-5-methoxymethylimidazole,
according to claim 1.

**3.** Imidazole derivatives of the general formula I according to claim 1, wherein $R^4$ is $COOR^5$,

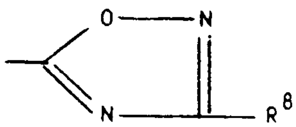

or

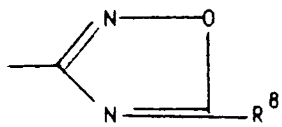

4. Medicaments based on the compounds according to claims 1 to 3.

5. Process for the preparation of the compounds of the formula I according to claim 1, characterised in that
a) an aniline of the general formula II

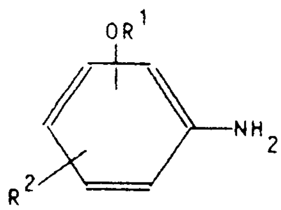

(II)

wherein
$R^1$ and $R^2$ have the meanings given in formula I, is reacted with a 2-azadiene of the general formula III

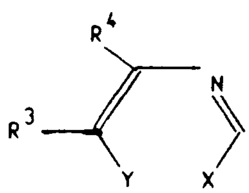

(III)

wherein
$R^3$ and $R^4$ have the meanings given in formula I and X and Y are leaving groups,
in the presence of an acid at temperatures of from 0 to 150 ° C, or
b) an imidazole derivative of the general formula IV

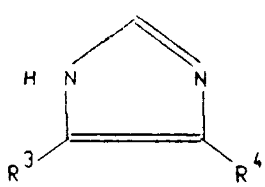

(IV)

wherein
$R^3$ and $R^4$ have the meanings given above, is arylated with an aromatic compound of the general formula V

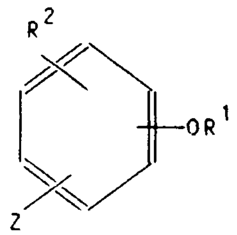

(V)

wherein
$R^1$ and $R^2$ have the meanings given above, and Z is a leaving group,
and optionally subsequently, in the compounds obtained according to a) or b), an ester group present in the molecule is transesterified or hydrolysed, a free carboxy group is optionally esterified, amidated or reacted with an amidoxime of the formula $R^8$-C(=NOH)$NH_2$ to form the 5-oxadiazolyl derivative, and optionally a nitrile group present in the molecule is hydrolysed to the carbonylamide or carboxy group or converted via the imino ester group into the ester group ($COOR^5$) or with hydroxylamine via the amidoxime and subsequently with an alkanecarboxylic acid of the formula $R^8$-COOH or an activated derivative of the acid into the 3-oxadiazolyl derivative and optionally compounds in which $R^1$ = H are etherified with the optionally substituted $C_{1-10}$hydrocarbon or hetaryl radical in the presence of a base, and optionally a nitro group is reduced to the amino group and the latter is then optionally alkylated or acylated or replaced by halogen, azide, cyano or thiocyanate.

**6.** Process for the manufacture of a medicament, characterised in that a compound of formula I prepared according to claim 5 is mixed with a pharmaceutical carrier.

## Revendications

**1.** Dérivés de l'imidazole répondant à la formule générale I :

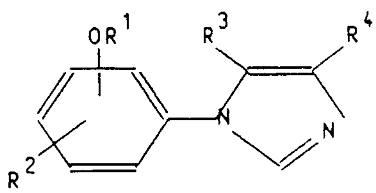

(I)

dans laquelle :

$R^1$ représente l'hydrogène, un radical hydrocarboné en $C_1$-$C_{10}$ ou un radical hétéro-aryle à 5 ou 6 maillons qui contient 1 ou 2 atomes de soufre, d'azote et/ou d'oxygène, chacun de ces radicaux hydrocarbonés et hétéro-aryles portant éventuellement de 1 à 3 substituants pris dans l'ensemble constitué par les halogènes, les radicaux nitro, cyano, hydroxy, mercapto, alkyles en $C_1$-$C_4$, alcoxy en $C_1$-$C_4$, alkylthio en $C_1$-$C_4$, alkylsulfinyles en $C_1$-$C_4$ et alkylsulfonyles en $C_1$-$C_4$ et par les radicaux amino éventuellement porteurs d'un ou deux substituants pris dans l'ensemble constitué par les alkyles en $C_1$-$C_4$, les alcanoyles en $C_1$-$C_4$ et les sulfonyles, ou $R^1$ représente un radical d'éther cyclique à 5 ou 6 maillons,

$R^2$ représente l'hydrogène, un halogène, un radical amino éventuellement porteur d'un ou de deux substituants pris dans l'ensemble constitué par les alkyles en $C_1$-$C_4$, les alcanoyles en $C_1$-$C_4$ et les sulfonyles, un radical nitro, azido, thiocyanato ou cyano, un radical alkyle en $C_1$-$C_{10}$ linéaire ou ramifié et éventuellement porteur d'un halogène, ou un radical -$OR^1$ dans lequel $R^1$ a la signification qui lui a été donnée ci-dessus, ou

$R^1$ et $R^2$ forment ensemble, et avec l'atome d'oxygène, un cycle saturé ou insaturé contenant de 5 à 7 maillons, cycle qui peut contenir un atome d'oxygène supplémentaire,

$R^3$ représente l'hydrogène, un alkyle en $C_1$-$C_6$ linéaire ou ramifié ou un radical $C_1$-$C_6$-

alcoxyalkyle, et

R4 représente un radical répondant à l'une des formules : $-COOR^5$, $-CONR^6R^7$, -CN,

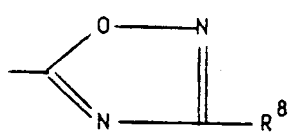

et

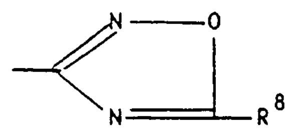

dans lesquelles $R^5$ représente l'hydrogène ou un alkyle en $C_1$-$C_6$ linéaire ou ramifié, $R^6$ et $R^7$ représentent chacun, indépendamment l'un de l'autre, l'hydrogène ou un radical alkyle, linéaire, ramifié ou cyclique, contenant au plus 7 atomes de carbone, ou forment ensemble, et avec l'atome d'azote, un radical de morpholine, de pipérazine, de pyrrolidine, de pipéridine ou de thiomorpholine, chacun de ces hétérocycles azotés pouvant porter un ou deux alkyles en $C_1$-$C_4$, et $R^8$ représente l'hydrogène ou un radical alkyle linéaire, ramifié ou cyclique qui contient au plus 7 atomes de carbone,

et $R^2$ peut exister en un ou en plusieurs exemplaires sur le radical phényle.

**2.** Composés selon la revendication 1, en l'espèce :
le 4-(3-éthyl-1,2,4-oxadiazol-5-yl)-1-(2-hydroxyphényl)-imidazole,
le 1-(2-benzyloxyphényl)-4-(3-éthyl-1,2,4-oxadiazol-5-yl)-imidazole,
le 1-[2-(4-chlorobenzyloxy)-phényl]-4-(3-éthyl-1,2,4-oxadiazol-5-yl)-imidazole
le 1-[2-(2-phényléthoxy)-phényl]-4-(3-éthyl-1,2,4-oxadiazol-5-yl)-imidazole
le 4-(3-éthyl-1,2,4-oxadiazol-5-yl)-1-(3-hydroxyphényl)-imidazole
le 1-(3-benzyloxyphényl)-4-(3-éthyl-1,2,4-oxadiazol-5-yl)-imidazole
le 1-[3-(4-chlorobenzyloxy)-phényl]-4-(3-éthyl-1,2,4-oxadiazol-5-yl)-imidazole
le 4-(3-éthyl-1,2,4-oxadiazol-5-yl)-1-[3-(2-phényléthoxy)-phényl]-imidazole
le 4-(3-éthyl-1,2,4-oxadiazol-5-yl)-1-[3-(4-nitrophénoxy)-phényl]-imidazole
le l-[3-(4-aminophénoxy)-phényl]-4-(3-éthyl-1,2,4-oxadiazol-5-yl)-imidazole
1-(4-benzyloxyphényl)-4-(3-éthyl-1,2,4-oxadiazol-5-yl)-imidazole
le 4-(3-éthyl-1,2,4-oxadiazol-5-yl)-1-(3-phénoxyphényl)-imidazole
le 1-[3-(4-chlorophénoxy)-phényl]-4-(3-éthyl-1,2,4-oxadiazol-5-yl)-imidazole
le 1-(5-chloro-2-méthoxyphényl)-4-(3-éthy1-1,2,4-oxadiazol-5-yl)-imidazole
le 1-(2-benzyloxy-5-chlorophényl)-4-(3-éthyl-1,2,4-oxadiazol-5-yl)-imidazole
le 1-(2-chloro-5-méthoxyphényl)-4-(3-éthyl-1,2,4-oxadiazol-5-yl)-imidazole
le 1-(3,4-méthylènedioxy-phényl)-4-(3-éthyl-1,2,4-oxadiazol-5-yl)-imidazole
le 1-(3,4-diméthoxyphényl)-imidazole-4-carboxylate d'éthyle
le 1-(3,4-diméthoxyphényl)-4-(3-éthyl-1,2,4-oxadiazol-5-yl)-1-imidazole
le 1-(2,6-dihydroxyphényl)-4-(3-éthyl-1,2,4-oxadiazol-5-yl)-imidazole
le 1-(2,6-dihydroxyphényl)-imidazole-4-carboxylate d'éthyle
le 1-(2,6-dibenzyloxyphényl)-imidazole-4-carboxylate d'éthyle
le 1-(2,6-dibenzyloxyphényl)-4-(3-éthyl-1,2,4-oxadiazol-5-yl)-imidazole
le 1-(3-méthoxyphényl)-imidazole-4-carboxylate d'éthyle
le 1-(3-méthoxyphényl)-imidazole-4-carboxylate d'isopropyle
le 4-(3-éthyl-1,2,4-oxadiazol-5-yl)-1-(3-méthoxyphényl)-imidazole
le 1-(3-éthoxyphényl)-4-(3-éthyl-1,2,4-oxadiazol-5-yl)-imidazole
le 1-(4-méthoxyphényl)-imidazole-4-carboxylate d'éthyle
le 4-(3-éthyl-1,2,4-oxadiazol-5-yl)-1-(4-méthoxyphényl)-imidazole
le 1-(4-phénoxyphényl)-imidazole-4-carboxylate d'éthyle
le 4-(3-éthyl-1,2,4-oxadiazol-5-yl)-1-(4-phénoxyphényl)-imidazole
le 4-(3-éthyl-1,2,4-oxadiazol-5-yl)-1-[3-(2-propényl)-oxyphényl]-imidazole
le 4-(3-éthyl-1,2,4-oxadiazol-5-yl)-1-(3-isopropyl-oxyphényl)-imidazole
le 1-(3-cyclopentyloxyphényl)-4-(3-éthyl-1,2,4-oxadiazol-5-yl)-imidazole
le [3-(5-bromo-2-pyridyloxy)-phényl]-4-(3-éthyl-1,2,4-oxadiazol-5-yl)-imidazole
le 1-(3-hydroxyphényl)-imidazole-4-carboxylate d'éthyle
le 1-(3-isopropyloxyphényl)-imidazole-4-carboxylate d'éthyle
le 1-(3-cyclopentyloxyphényl)-imidazole-4-carboxylate d'éthyle

le 1-[3-(2-propényloxy)-phényl]-imidazole-4-carboxylate d'éthyle
le 1-[3-(5-bromo-2-pyridyloxy)-phényl]-imidazole-4-carboxylate d'éthyle
le 1-[3-(4-nitrophénoxy)-phényl]-imidazole-4-carboxylate d'éthyle
le 1-[3-(4-aminophénoxy)-phényl]-imidazole-4-carboxylate d'éthyle
le 1-[3-(4-chlorphénoxy)-phényl]-imidazole-4-carboxylate d'éthyle
le 1-(3-cyclopentyloxyphényl)-imidazole-4-carboxylate d'éthyle
le 1-(3-benzyloxyphényl)-imidazole-4-carbonitrile
le 1-(3-hydroxyphényl)-5-méthyl-imidazole-4-carbonitrile
le 4-(5-éthyl-1,2,4-oxadiazol-3-yl)-1-(3-hydroxyphényl)-5-méthyl-imidazole
le 1-(3-cyclopentyloxy-phényl)-4-(5-éthyl-1,2,4-oxadiazol-3-yl)-5-méthyl-imidazole
le 5-méthyl-1-(3-phénoxyphényl)-imidazole-4-carbonitrile
le 4-(5-éthyl-1,2,4-oxadiazol-3-yl)-5-méthyl-1-(3-phénoxyphényl)-imidazole
le 1-(3-benzyloxyphényl)-4-(3-éthyl-1,2,4-oxadiazole-5-yl)-5-méthyl-imidazole
le 4-(3-éthyl-1,2,4-oxadiazol-5-yl)-1-(3-hydroxyphényl)-5-méthoxyméthyl-imidazole
le 4-(3-éthyl-1,2,4-oxadiazol-5-yl)-5-méthoxyméthyl-1-(3-méthoxyphényl)-imidazole
le 1-[3-(4-chlorophénoxy)-phényl]-4-(3-éthyl-1,2,4-oxadiazol-5-yl)-5-méthoxyméthyl-imidazole
le 1-(3-benzyloxy-phényl)-4-(3-éthyl-1,2,4-oxadiazol-5-yl)-5-méthoxyméthyl-imidazole
le 1-(3-cyclopentyloxy-phényl)-4-(3-éthyl-1,2,4-oxadiazol-5-yl)-5-méthoxyméthyl-imidazole
le 4-(3-éthyl-1,2,4-oxadiazol-5-yl)-5-méthoxyméthyl-1-[3-(4-nitrophénoxy)-phényl]-imidazole et
le 1-[3-(4-chlorobenzyloxy)-phényl]-4-(3-éthyl-1,2,4-oxadiazol-5-yl)-5-méthoxyméthyl-imidazole.

**3.** Dérivés de l'imidazole de formule générale I selon la revendication 1, dans lesquels $R^4$ représente un radical $-COOR^5$,

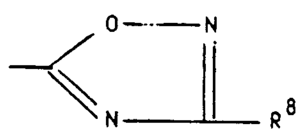

ou

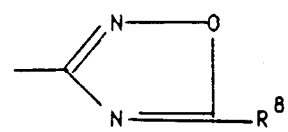

**4.** Médicaments à base de composés selon l'une quelconque des revendications 1 à 3.

**5.** Procédé pour préparer les composés de formule I selon la revendication 1, procédé caractérisé en ce que :

a) on fait réagir une aniline répondant à la formule générale II :

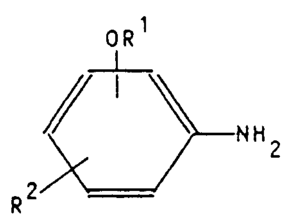

(II) ;

dans laquelle $R^1$ et $R^2$ ont les significations qui leur ont été données à propos de la formule I, avec un 2-azadiène répondant à la formule générale III :

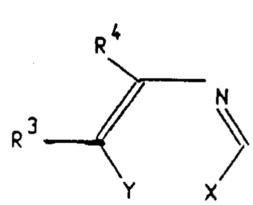

(III)

dans laquelle $R^3$ et $R^4$ ont les significations qui leur ont été données à propos de la formule I, et X

et Y représentent des radicaux éliminables,
en présence d'acides, à des températures de 0 à 150 ° C, ou
b) on aryle un dérivé de l'imidazole répondant à la formule générale IV :

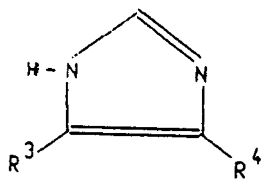

(IV)

dans laquelle $R^3$ et $R^4$ ont les significations qui leur ont été données ci-dessus, avec un composé aromatique répondant à la formule générale V :

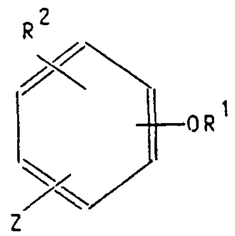

(V)

dans laquelle $R^1$ et $R^2$ ont les significations qui leur ont été données ci-dessus et Z représente un radical éliminable,
après quoi, le cas échéant, dans les composés obtenus selon a) ou b), on transestérifie ou saponifie un radical ester si la molécule en contient un, on estérifie éventuellement ou convertit en amide un radical carboxy libre ou encore on le fait réagir avec une amide-oxime de formule $R^8$-C(=NOH)$NH^2$ pour le convertir en un radical 5-oxa-diazolyle, et éventuellement on hydrolyse un radical nitrile présent dans la molécule pour le convertir en un radical carbamoyle ou carboxy ou on le convertit, en passant par le radical d'ester carboximidique, en le radical ester (-$COOR^5$) ou on le convertit, par réaction avec l'hydroxylamine, en passant par l'amide-oxime, puis réaction avec un acide alcanoïque de formule $R^8$-COOH ou un dérive activé d'un tel acide, en le radical 3-oxadiazolyle, et éventuellement on éthérifie des composés dans lesquels $R^1$ représente H, en présence de bases, avec un radical hydrocarboné en $C_1$-$C_{10}$ ou un radical hétaryle, éventuellement substitués, et éventuellement on réduit un radical nitro en un radical amino, puis, éventuellement, on alkyle ou acyle celui-ci ou on l'échange contre un halogène ou un radical azido, cyano ou thiocyanato.

**6.** Procédé pour préparer un médicament, procédé caractérisé en ce qu'on mélange un composé de formule I qui a été préparé selon la revendication 5 avec un excipient pharmaceutique.